# EUROPEAN PATENT SPECIFICATION

(11) **EP 3 555 102 B1**
(45) Date of publication and mention of the grant of the patent: **20.01.2021**
(21) Application number: 17821867.3
(22) Date of filing: 14.12.2017
(51) Int. Cl.: C07D 493/04, A01N 43/12

(54) **HERBICIDAL COMPOUNDS**
HERBIZIDE VERBINDUNGEN
COMPOSÉS HERBICIDES

(30) Priority: 19.12.2016 GB 201621626
(43) Date of publication of application: 23.10.2019
(73) Proprietor: Syngenta Participations AG, 4058 Basel (CH)
(72) Inventor: HENNESSY, Alan, Joseph, Bracknell Berkshire RG42 6EY (GB); HACHISU, Shuji, Bracknell GB RG42 6EY (GB); WILLETTS, Nigel, James, Bracknell Berkshire RG42 6EY (GB); DALE, Suzanna, Jane, Bracknell Berkshire RG42 6EY (GB)
(74) Representative: SYNGENTA IP
(86) International application number: PCT/EP2017/082798
(87) International publication number: WO 2018/114584

(56) References cited:
- WO-A1-2009/019015
- WO-A1-2013/079708
- WO-A1-2014/191534

## Description

The present invention relates to novel cyclopentanedione herbicidal compounds, to processes for their preparation, to herbicidal compositions which comprise the novel compounds, and to their use for controlling weeds.

Herbicidal bicyclic1,3-diones are disclosed in, for example, WO2009/019015, WO2013/079708 and WO2014/191534. The present invention relates to novel herbicidal cyclopentanedione derivatives with improved properties.

Thus, according to the present invention there is provided a compound of Formula (I): 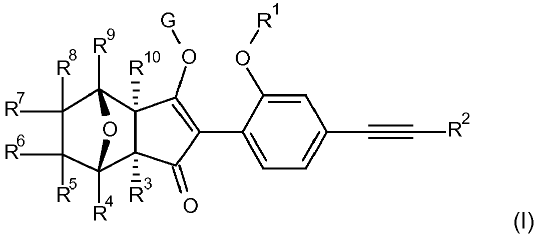 wherein
G is selected from the group consisting of hydrogen, -(CH₂)ₙ-R^{a}, -C(O)-R^{a},-C(O)-O-R^{d}, -C(O)NR^{a}R^{a}, -S(O)₂-C₁-C₈alkyl and -C₁-C₃alkoxyC₁-C₈alkyl;
R^{a} is independently selected from the group consisting of hydrogen, C₁₋C₈alkyl, C₁-C₃haloalkyl, C₂-C₈alkenyl, C₂-C₈alkynyl, C₃-C₆ cycloalkyl and phenyl;
R^{d} is independently selected from the group consisting of C₁-C₈alkyl, C₁-C₃haloalkyl, C₂-C₈alkenyl, C₂-C₈alkynyl, C₃-C₆ cycloalkyl and phenyl;
R¹ is selected from the group consisting of C₁-C₃alkyl, C₁-C₃alkoxyC₁-C₃alkyl- and C₁-C₃haloalkyl;
R² is C₁-C₃alkyl;
R³ and R¹⁰ are independently selected from the group consisting of hydrogen and C₁-C₃alkyl;
R⁴ and R⁹ are independently selected from the group consisting of hydrogen, C₁-C₃alkyl and C₁-C₃alkoxyC₁-C₃alkyl;
R⁶ and R⁷ are independently selected from the group consisting of hydrogen, halogen, -(CH₂)ₙ-OH, cyano, C₁-C₆alkyl, C₃-C₆cycloalkyl-, C₁-C₆haloalkyl, C₂-C₆alkenyl, C₂-C₆alkynyl, C₁-C₆alkoxy, C₂-C₆alkenyloxy-, C₂-C₆alkynyloxy-, C₁-C₆alkoxyC₁-C₆alkyl-, C₁-C₆alkoxyC₁-C₆alkoxy-, -O-C(O)C₁-C₆alkyl, - CH₂OCH₂CN, -CH=NOH, -CH=NO-C₁-C₃alkyl, -C(CH₃)=NOH, -C(CH₃)=NO-C₁-C₃alkyl, -CH₂OC(O)NHC₁-C₆alkyl, -(CH₂)ₙNR^{b}R^{c}, -C(O)NR^{b}R^{c}, - (CH₂)ₙNHC(O)H, -(CH₂)ₙNHC(O)C₁-C₆alkyl, -(CH₂)ₙNHC(O)OC₁-C₆alkyl, - NHC(O)NHC(O)C₁-C₆alkyl, -(CH₂)ₙ-N(R^{b})OR^{c}, -NHC(O)NR^{b}R^{c}, C₁-C₆haloalkoxy-, C₂-C₆alkenoxyC₁-C₆alkyl-, C₂-C₆alkynyloxyC₁-C₆alkyl-, C₁-C₆haloalkoxyC₁-C₆alkyl-, aryl, heteroaryl, and a 5 or 6-membered saturated or partially unsaturated ring system wherein the aryl, heteroaryl and ring system are optionally substituted by one or two independent R¹¹;
R^{b} and R^{c} are independently selected from the group consisting of hydrogen, phenyl and C₁-C₆alkyl; and
R⁵ and R⁸ form a bond or are independently selected from the group consisting of hydrogen, halogen, cyano, C₁-C₆alkyl, C₂-C₆alkenyl, C₂-C₆alkynyl, C₁-C₆alkoxy, C₁-C₆alkoxyC₁-C₆alkyl- and C₁-C₆alkoxyC₁-C₆alkoxy-; or
R⁵ and R⁶ together form =O, =NOH, =NOC₁-C₃alkyl, -X⁴-CH₂-CH₂-X⁵- or -X⁴-CH₂-CH₂-CH₂-X⁵- wherein X⁴ is CH₂ or O and X⁵ is CH₂, O or NH; and R⁷ and R⁸ are independently selected from the group consisting of hydrogen, halogen, cyano, C₁-C₆alkyl, C₂-C₆alkenyl, C₂-C₆alkynyl, C₁-C₆alkoxy-, C₁-C₆alkoxyC₁-C₆alkyl- and C₁-C₆alkoxyC₁-C₆alkoxy-; or
R⁷ and R⁸ together form =O, =NOH, =NOC₁-C₃alkyl, -X⁴-CH₂-CH₂-X⁵- or -X⁴-CH₂-CH₂-CH₂-X⁵- wherein X⁴ is CH₂ or O and X⁵ is CH₂, O or NH; and R⁵ and R⁶ are independently selected from the group consisting of hydrogen, halogen, cyano, C₁-C₆alkyl, C₂-C₆alkenyl, C₂-C₆alkynyl, C₁-C₆alkoxy, C₁-C₆alkoxyC₁-C₆alkyl- and C₁-C₆alkoxyC₁-C₆alkoxy-; and
R¹¹ is selected from the group consisting of C₁-C₃alkyl, C₁-C₃haloalkyl-, C₁-C₃alkoxy-, C₁-C₃haloalkoxy-, cyano and halogen; and
n = 0, 1 or 2;
or an agriculturally acceptable salt thereof.

Alkyl groups (e.g C₁-C₆alkyl) include, for example, methyl (Me, CH₃), ethyl (Et, C₂H₅), *n*-propyl (*n*-Pr), isopropyl (*i*-Pr), *n*-butyl (*n*-Bu), isobutyl (*i*-Bu), *sec*-butyl (*s*-Bu) and *tert*-butyl (*t*-Bu).

Alkenyl and alkynyl moieties can be in the form of straight or branched chains, and the alkenyl moieties, where appropriate, can be of either the (*E*)- or (*Z*)-configuration. Examples are vinyl, allyl and propargyl. Alkenyl and alkynyl moieties can contain one or more double and/or triple bonds in any combination.

Halogen (or halo) encompasses fluorine, chlorine, bromine or iodine. The same correspondingly applies to halogen in the context of other definitions, such as haloalkyl.

Haloalkyl groups (e.g C₁-C₆haloalkyl) are, for example, fluoromethyl, difluoromethyl, trifluoromethyl, chloromethyl, dichloromethyl, trichloromethyl, 2,2,2-trifluoroethyl, 2-fluoroethyl, 2-chloroethyl, pentafluoroethyl, 1,1-difluoro-2,2,2-trichloroethyl, 2,2,3,3-tetrafluoroethyl and 2,2,2-trichloroethyl, heptafluoro-n-propyl and perfluoro-n-hexyl.

Alkoxy groups (e.g C₁-C₆alkoxy) are, for example, methoxy, ethoxy, propoxy, isopropoxy, n-butoxy, isobutoxy, sec-butoxy or tert-butoxy or a pentyloxy or hexyloxy isomer, preferably methoxy and ethoxy. It should also be appreciated that two alkoxy substituents present on the same carbon atom may be joined to form a spiro group. Thus, the methyl groups present in two methoxy substituents may be joined to form a spiro 1,3-dioxolane substituent, for example. Such a possibility is within the scope of the present invention.

Alkoxyalkyl groups (e.g C₁-C₆alkoxyC₁-C₆alkyl-) includes, for example, methoxymethyl, methoxyethyl, ethoxymethyl, ethoxyethyl, n-propoxymethyl, n-propoxyethyl, isopropoxymethyl or isopropoxyethyl.

Cycloalkyl groups (e.g C₃-C₆cycloalkyl-) include, for example cyclopropyl (*c-*propyl, *c*-Pr), cyclobutyl (*c*-butyl, *c*-Bu), cyclopentyl (*c*-pentyl) and cyclohexyl (*c*-hexyl) and may be substituted or unsubstituted as indicated.

The invention also relates to agriculturally acceptable salts of the compounds of Formula (I). Such salts include those which are able to form with amines, alkali metal and alkaline earth metal bases or quaternary ammonium bases. Among the alkali metal and alkaline earth metal hydroxides as salt formers, special mention should be made of the hydroxides of lithium, sodium, potassium, magnesium and calcium, but especially the hydroxides of sodium and potassium. The compounds of Formula (I) according to the invention also include hydrates which may be formed during the salt formation.

Examples of amines suitable for ammonium salt formation include ammonia as well as primary, secondary and tertiary C₁-C₁₈alkylamines, C₁-C₄hydroxyalkylamines and C₂-C₄alkoxyalkylamines, for example methylamine, ethylamine, n-propylamine, isopropylamine, the four butylamine isomers, n-amylamine, isoamylamine, hexylamine, heptylamine, octylamine, nonylamine, decylamine, pentadecylamine, hexadecylamine, heptadecylamine, octadecylamine, methylethylamine, methylisopropylamine, methylhexylamine, methylnonylamine, methylpentadecylamine, methyloctadecylamine, ethylbutylamine, ethylheptylamine, ethyloctylamine, hexylheptylamine, hexyloctylamine, dimethylamine, diethylamine, di*n*-propylamine, diisopropylamine, di-*n*-butylamine, di-*n*-amylamine, diisoamylamine, dihexylamine, diheptylamine, dioctylamine, ethanolamine, *n*-propanolamine, isopropanolamine, *N*,*N*-diethanolamine, *N*-ethylpropanolamine, *N*-butylethanolamine, allylamine, *n*-but-2-enylamine, *n*-pent-2-enylamine, 2,3-dimethylbut-2-enylamine, dibut-2-enylamine, n-hex-2-enylamine, propylenediamine, trimethylamine, triethylamine, tri-*n*-propylamine, triisopropylamine, tri-*n*-butylamine, triisobutylamine, tri-*sec*-butylamine, tri-*n*-amylamine, methoxyethylamine and ethoxyethylamine; heterocyclic amines, for example pyridine, quinoline, isoquinoline, morpholine, piperidine, pyrrolidine, indoline, quinuclidine and azepine; primary arylamines, for example anilines, methoxyanilines, ethoxyanilines, *o*-, *m*- and *p*-toluidines, phenylenediamines, benzidines, naphthylamines and *o*-, *m*- and *p*-chloroanilines; but especially triethylamine, isopropylamine and diisopropylamine.

In one embodiment of the present invention, G is selected from the group consisting of hydrogen, C₁-C₈alkyl (e.g methyl, ethyl, *n*-propyl, *i*-propyl, *n*-butyl, *t*-butyl, -C₂-C₈alkenyl (e.g vinyl), C₂-C₈alkynyl (e.g propargyl), -C(O)C₁-C₈alkyl (e.g -C(O)*i-*propyl and -C(O)*t*-butyl). In a preferred embodiment, G is hydrogen.

In one embodiment of the present invention R¹ is methyl.

In one embodiment of the present invention, R² is methyl.

In one embodiment of the present invention, R³ and R¹⁰ are independently selected from the group consisting of hydrogen, methyl and ethyl. In a preferred embodiment of the present invention, R³ and R¹⁰ are both hydrogen.

In one embodiment of the present invention, R⁴ and R⁹ are independently selected from the group consisting of hydrogen, methyl, ethyl and methoxymethyl-. In a preferred embodiment, R⁴ and R⁹ are both hydrogen.

In one embodiment of the present invention R⁶ and R⁷ are independently selected from the group consisting of hydrogen, cyano, C₁-C₆alkyl (e.g methyl, ethyl), C₂-C₆alkenyl (e.g vinyl), C₂-C₆alkynyl (e.g propargyl), C₁-C₆alkoxy (e.g methoxy-), C₁-C₆alkoxyC₁-C₆alkyl (e.g methoxymethyl-) and C₁-C₆alkoxyC₁-C₆alkoxy- (e.g methoxyethoxy). In another embodiment, R⁵ and R⁸ may also be selected from the group consisting of aryl (e.g phenyl), heteroaryl (e.g pyridyl) and a 5- or 6-membered saturated or partially unsaturated ring system (e.g tetrahydropyranyl-, 1,3 dioxolanyl, isoxazolyl). In a preferred embodiment, the 5- or 6-membered saturated or partially unsaturated ring system is selected from the group consisting of A1, A2 and A3: 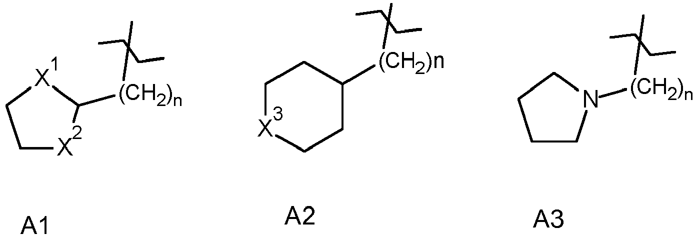 wherein X¹, X² and X³ are independently selected from the group consisting of O, C(R¹²R¹³), N-(O-C₁-C₃alkyl), N-(CO)-C₁-C₃alkyl and N-(CO)O-C₁-C₃alkyl, and wherein R¹² and R¹³ are independently hydrogen or C₁-C₆ alkyl; and

R⁵ and R⁸ form a bond or are independently selected from the group consisting of hydrogen, cyano, C₁-C₆alkyl (e.g methyl, ethyl), C₂-C₆alkenyl (e.g vinyl), C₂-C₆alkynyl (e.g propargyl), C₁-C₆alkoxy (e.g methoxy-), C₁-C₆alkoxyC₁-C₆alkyl (e.g methoxymethyl-) and C₁-C₆alkoxyC₁-C₆alkoxy- (e.g methoxyethoxy).

In a preferred embodiment, R⁵, R⁶, R⁷ and R⁸ are selected from the group consisting of methyl, ethyl, methoxymethyl- and methoxy. In one embodiment, R⁵, R⁶, R⁷ and R⁸ are all hydrogen.

In a preferred embodiment of the present invention there is provided a compound of Formula I wherein R⁴, R⁵, R⁸ and R⁹ are all hydrogen and, and one of R⁶ and R⁷ are C₁-C₆alkoxyC₁-C₆alkyl-.

In another embodiment of the present invention there is provided a compound of Formula I wherein R³, R⁴, R⁵, R⁸, R⁹ and R¹⁰ are all hydrogen, and one of R⁶ and R⁷ are C₁-C₆alkoxyC₁-C₆alkyl-, e.g methoxymethyl.

In another embodiment of the present invention there is provided a compound of Formula (I) wherein R¹ is C₁-C₃alkyl (preferably methyl), R² is C₁-C₃alkyl (preferably methyl), R³, R⁴, R⁵, R⁸, R⁹ and R¹⁰ are hydrogen and
R⁶ is hydrogen and R⁷, is methoxymethyl-; or
R⁶ is methoxymethyl- and R⁷ is hydrogen.

In another embodiment of the present invention, R⁵ and R⁶ together form =O, =NOH, =NOC₁-C₃alkyl, -X⁴-CH₂-CH₂-X⁵- or -X⁴-CH₂-CH₂-CH₂-X⁵- wherein X⁴ is CH₂ or O and X⁵ is CH₂, O or NH; and R⁷ and R⁸ are independently selected from the group consisting of hydrogen, halogen, cyano, C₁-C₆alkyl, C₂-C₆alkenyl, C₂-C₆alkynyl, C₁-C₆alkoxy-, C₁-C₆alkoxyC₁-C₆alkyl- and C₁-C₆alkoxyC₁-C₆alkoxy-. In a preferred embodiment R⁵ and R⁶ form -O-CH₂-CH₂-O- or -O-CH₂-CH₂-CH₂-O-.

In another embodiment of the present invention, R⁷ and R⁸ together form =O, =NOH, =NOC₁-C₃alkyl, -X⁴-CH₂-CH₂-X⁵- or -X⁴-CH₂-CH₂-CH₂-X⁵- wherein X⁴ is CH₂ or O and X⁵ is CH₂, O or NH; and R⁵ and R⁶ are independently selected from the group consisting of hydrogen, halogen, cyano, C₁-C₆alkyl, C₂-C₆alkenyl, C₂-C₆alkynyl, C₁-C₆alkoxy, C₁-C₆alkoxyC₁-C₆alkyl- and C₁-C₆alkoxyC₁-C₆alkoxy-. In a preferred embodiment R⁷ and R⁸ form -O-CH₂-CH₂-O- or -O-CH₂-CH₂-CH₂-O-.

In another embodiment of the present invention, R⁵ and R⁸ form a bond to give a compound of Formula (Ia): 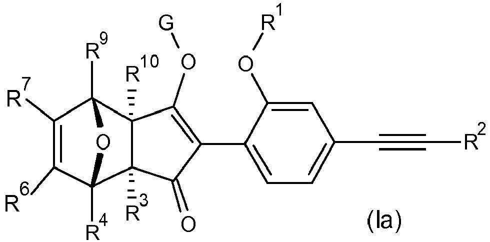 wherein R¹, R², R³, R⁴, R⁶, R⁷, R⁹, R¹⁰ and G are as defined above. With regard to compounds of Formula (Ia), a preferred embodiment is wherein R³, R⁴, R⁶, R⁹ and R¹⁰ are hydrogen, and R⁷ is methoxymethyl-. In another embodiment of the present invention, there is provided a compound of Formula (Ia), wherein R³, R⁴, R⁷, R⁹ and R¹⁰ are hydrogen, and R⁶ is methoxymethyl-.

In another embodiment of the present invention is a compound of Formula I wherein R⁴ is methyl, R⁹ is methyl and R³, R⁵, R⁶, R⁷, R⁸ and R¹⁰ are all hydrogen.

In another embodiment of the present invention is a compound of Formula I wherein R⁴ is ethyl, R⁹ is ethyl and R³, R⁵, R⁶, R⁷, R⁸ and R¹⁰ are all hydrogen.

In another embodiment of the present invention is a compound of Formula I wherein R⁴ is methoxy and R⁹ is methoxy and R³, R⁵, R⁶, R⁷, R⁸ and R¹⁰ are all hydrogen.

In another embodiment of the present invention is a compound of Formula I wherein R⁶ is methyl, R⁷ is methyl and R³, R⁴, R⁵, R⁸, R⁹ and R¹⁰ are all hydrogen.

In another embodiment of the present invention is a compound of Formula I wherein R⁶ is ethyl, R⁷ is ethyl and R³, R⁴, R⁵, R⁸, R⁹ and R¹⁰ are all hydrogen.

In another embodiment of the present invention is a compound of Formula I wherein R⁶ is methoxy and R⁷ is methoxy and R³, R⁴, R⁵, R⁸, R⁹ and R¹⁰ are all hydrogen.

In another embodiment of the present invention is a compound of Formula I wherein R⁶ is methyl and R³, R⁴, R⁵, R⁷, R⁸, R⁹ and R¹⁰ are all hydrogen.

In another embodiment of the present invention is a compound of Formula I wherein R⁶ is ethyl and R³, R⁴, R⁵, R⁷, R⁸, R⁹ and R¹⁰ are all hydrogen.

In another embodiment of the present invention is a compound of Formula I wherein R⁶ is methoxy and R³, R⁴, R⁵, R⁷, R⁸, R⁹ and R¹⁰ are all hydrogen.

In another embodiment of the present invention is a compound of Formula I wherein R⁹ is methyl and R³, R⁴, R⁵, R⁶, R⁷, R⁸, and R¹⁰ are all hydrogen.

In another embodiment of the present invention is a compound of Formula I wherein R⁹ is ethyl and R³, R⁴, R⁵, R⁶, R⁷, R⁸, and R¹⁰ are all hydrogen.

In another embodiment of the present invention is a compound of Formula I wherein R⁹ is methoxy and R³, R⁴, R⁵, R⁶, R⁷, R⁸, and R¹⁰ are all hydrogen.

In another embodiment of the present invention is a compound of Formula I wherein R³, R⁴, R⁵, R⁶, R⁷, R⁸, R⁹ and R¹⁰ are all hydrogen.

Depending on the nature of the substituents, compounds of Formula (I) may exist in different isomeric forms. When G is hydrogen, for example, compounds of Formula (I) may exist in different tautomeric forms.

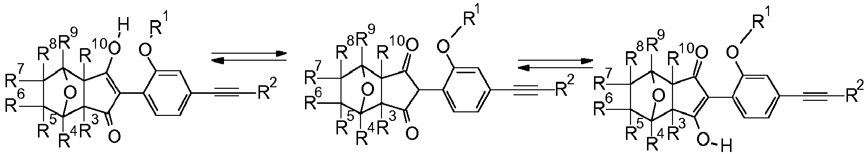

This invention covers all such isomers and tautomers and mixtures thereof in all proportions. Also, when substituents contain double bonds, *cis*- and trans-isomers can exist. These isomers, too, are within the scope of the claimed compounds of the Formula (I). Compounds of Formula (I) may contain asymmetric centres and may be present as a single enantiomer, pairs of enantiomers in any proportion or, where more than one asymmetric centre are present, contain diastereoisomers in all possible ratios. Typically one of the enantiomers has enhanced biological activity compared to the other possibilities.

The compounds of Formula (I) according to the invention can be used as herbicides by themselves, but they are generally formulated into herbicidal compositions using formulation adjuvants, such as carriers, solvents and surface-active agents (SFAs). Thus, the present invention further provides a herbicidal composition comprising a herbicidal compound according to any one of the previous claims and an agriculturally acceptable formulation adjuvant. The composition can be in the form of concentrates which are diluted prior to use, although ready-to-use compositions can also be made. The final dilution is usually made with water, but can be made instead of, or in addition to, water, with, for example, liquid fertilisers, micronutrients, biological organisms, oil or solvents.

The herbicidal compositions generally comprise from 0.1 to 99 % by weight, especially from 0.1 to 95 % by weight, compounds of Formula (I) and from 1 to 99.9 % by weight of a formulation adjuvant which preferably includes from 0 to 25 % by weight of a surface-active substance.

The compositions can be chosen from a number of formulation types, many of which are known from the Manual on Development and Use of FAO Specifications for Plant Protection Products, 5th Edition, 1999. These include dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, capsule suspensions (CS) and seed treatment formulations. The formulation type chosen in any instance will depend upon the particular purpose envisaged and the physical, chemical and biological properties of the compound of Formula (I).

Dustable powders (DP) may be prepared by mixing a compound of Formula (I) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of Formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of Formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of Formula (I) and one or more powdered solid diluents or carriers, or from preformed blank granules by absorbing a compound of Formula (I) (or a solution thereof, in a suitable agent) in a porous granular material (such as pumice, attapulgite clays, fuller's earth, kieselguhr, diatomaceous earths or ground corn cobs) or by adsorbing a compound of Formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of Formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of Formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone) and alcohols (such as benzyl alcohol, furfuryl alcohol or butanol), N-alkylpyrrolidones (such as N-methylpyrrolidone or N-octylpyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment.

Preparation of an EW involves obtaining a compound of Formula (I) either as a liquid (if it is not a liquid at room temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents which have a low solubility in water.

Microemulsions (ME) may be prepared by mixing water with a blend of one or more solvents with one or more SFAs, to produce spontaneously a thermodynamically stable isotropic liquid formulation. A compound of Formula (I) is present initially in either the water or the solvent/SFA blend. Suitable solvents for use in MEs include those hereinbefore described for use in in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of Formula (I). SCs may be prepared by ball or bead milling the solid compound of Formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of Formula (I) may be dry milled and added to water, containing agents hereinbefore described, to produce the desired end product.

Aerosol formulations comprise a compound of Formula (I) and a suitable propellant (for example n-butane). A compound of Formula (I) may also be dissolved or dispersed in a suitable medium (for example water or a water miscible liquid, such as n-propanol) to provide compositions for use in non-pressurised, hand-actuated spray pumps.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of Formula (I) and, optionally, a carrier or diluent therefor. The polymeric shell may be produced by either an interfacial polycondensation reaction or by a coacervation procedure. The compositions may provide for controlled release of the compound of Formula (I) and they may be used for seed treatment. A compound of Formula (I) may also be formulated in a biodegradable polymeric matrix to provide a slow, controlled release of the compound.

The composition may include one or more additives to improve the biological performance of the composition, for example by improving wetting, retention or distribution on surfaces; resistance to rain on treated surfaces; or uptake or mobility of a compound of Formula (I). Such additives include surface active agents (SFAs), spray additives based on oils, for example certain mineral oils or natural plant oils (such as soy bean and rape seed oil), and blends of these with other bio-enhancing adjuvants (ingredients which may aid or modify the action of a compound of Formula (I).

Wetting agents, dispersing agents and emulsifying agents may be SFAs of the cationic, anionic, amphoteric or non-ionic type.

Suitable SFAs of the cationic type include quaternary ammonium compounds (for example cetyltrimethyl ammonium bromide), imidazolines and amine salts.

Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butylnaphthalene sulphonate and mixtures of sodium di-*iso*propyl- and tri-*iso*propyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefine sulphonates, taurates and lignosulphonates.

Suitable SFAs of the amphoteric type include betaines, propionates and glycinates.

Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

The composition of the present may further comprise at least one additional pesticide. For example, the compounds according to the invention can also be used in combination with other herbicides or plant growth regulators. In a preferred embodiment the additional pesticide is a herbicide and/or herbicide safener. Examples of such mixtures are (in which 'I' represents a compound of Formula (I)). I + acetochlor, I + acifluorfen, I + acifluorfen-sodium, I + aclonifen, I + acrolein, I + alachlor, I + alloxydim, I + ametryn, I + amicarbazone, I + amidosulfuron, I + aminopyralid, I + amitrole, I + anilofos, I + asulam, I + atrazine, I + azafenidin, I + azimsulfuron, I + BCPC, I + beflubutamid, I + benazolin, I + bencarbazone, I + benfluralin, I + benfuresate, I + bensulfuron, I + bensulfuron-methyl, I + bensulide, I + bentazone, I + benzfendizone, I + benzobicyclon, I + benzofenap, I + bicyclopyrone, I + bifenox, I + bilanafos, I + bispyribac, I + bispyribac-sodium, I + borax, I + bromacil, I + bromobutide, I + bromoxynil, I + butachlor, I + butamifos, I + butralin, I + butroxydim, I + butylate, I + cacodylic acid, I + calcium chlorate, I + cafenstrole, I + carbetamide, I + carfentrazone, I + carfentrazone-ethyl, I + chlorflurenol, I + chlorflurenol-methyl, I + chloridazon, I + chlorimuron, I + chlorimuron-ethyl, I + chloroacetic acid, I + chlorotoluron, I + chlorpropham, I + chlorsulfuron, I + chlorthal, I + chlorthal-dimethyl, I + cinidon-ethyl, I + cinmethylin, I + cinosulfuron, I + cisanilide, I + clethodim, I + clodinafop, I + clodinafop-propargyl, I + clomazone, I + clomeprop, I + clopyralid, I + cloransulam, I + cloransulam-methyl, I + cyanazine, I + cycloate, I + cyclosulfamuron, I + cycloxydim, I + cyhalofop, I + cyhalofop-butyl,, I + 2,4-D, I + daimuron, I + dalapon, I + dazomet, I + 2,4-DB, I + I + desmedipham, I + dicamba, I + dichlobenil, I + dichlorprop, I + dichlorprop-P, I + diclofop, I + diclofop-methyl, I + diclosulam, I + difenzoquat, I + difenzoquat metilsulfate, I + diflufenican, I + diflufenzopyr, I + dimefuron, I + dimepiperate, I + dimethachlor, I + dimethametryn, I + dimethenamid, I + dimethenamid-P, I + dimethipin, I + dimethylarsinic acid, I + dinitramine, I + dinoterb, I + diphenamid, I + dipropetryn, I + diquat, I + diquat dibromide, I + dithiopyr, I + diuron, I + endothal, I + EPTC, I + esprocarb, I + ethalfluralin, I + ethametsulfuron, I + ethametsulfuron-methyl, I + ethephon, I + ethofumesate, I + ethoxyfen, I + ethoxysulfuron, I + etobenzanid, I + fenoxaprop-P, I + fenoxaprop-P-ethyl, I + fenquinotrione, I + fentrazamide, I + ferrous sulfate, I + flamprop-M, I + flazasulfuron, I + florasulam, I + fluazifop, I + fluazifop-butyl, I + fluazifop-P, I + fluazifop-P-butyl, I + fluazolate, I + flucarbazone, I + flucarbazone-sodium, I + flucetosulfuron, I + fluchloralin, I + flufenacet, I + flufenpyr, I + flufenpyr-ethyl, I + flumetralin, I + flumetsulam, I + flumiclorac, I + flumiclorac-pentyl, I + flumioxazin, I + flumipropin, I + fluometuron, I + fluoroglycofen, I + fluoroglycofen-ethyl, I + fluoxaprop, I + flupoxam, I + flupropacil, I + flupropanate, I + flupyrsulfuron, I + flupyrsulfuron-methyl-sodium, I + flurenol, I + fluridone, I + flurochloridone, I + fluroxypyr, I + flurtamone, I + fluthiacet, I + fluthiacet-methyl, I + fomesafen, I + foramsulfuron, I + fosamine, I + glufosinate, I + glufosinate-ammonium, I + glyphosate, I + halauxifen, I + halosulfuron, I + halosulfuron-methyl, I + haloxyfop, I + haloxyfop-P, I + hexazinone, I + imazamethabenz, I + imazamethabenz-methyl, I + imazamox, I + imazapic, I + imazapyr, I + imazaquin, I + imazethapyr, I + imazosulfuron, I + indanofan, I + indaziflam, I + iodomethane, I + iodosulfuron, I + iodosulfuron-methyl-sodium, I + ioxynil, I + isoproturon, I + isouron, I + isoxaben, I + isoxachlortole, I + isoxaflutole, I + isoxapyrifop, I + karbutilate, I + lactofen, I + lenacil, I + linuron, I + mecoprop, I + mecoprop-P, I + mefenacet, I + mefluidide, I + mesosulfuron, I + mesosulfuron-methyl, I + mesotrione, I + metam, I + metamifop, I + metamitron, I + metazachlor, I + methabenzthiazuron, I + methazole, I + methylarsonic acid, I + methyldymron, I + methyl isothiocyanate, I + metolachlor, I + S-metolachlor, I + metosulam, I + metoxuron, I + metribuzin, I + metsulfuron, I + metsulfuron-methyl, I + molinate, I + monolinuron, I + naproanilide, I + napropamide, I + naptalam, I + neburon, I + nicosulfuron, I + n-methyl glyphosate, I + nonanoic acid, I + norflurazon, I + oleic acid (fatty acids), I + orbencarb, I + orthosulfamuron, I + oryzalin, I + oxadiargyl, I + oxadiazon, I + oxasulfuron, I + oxaziclomefone, I + oxyfluorfen, I + paraquat, I + paraquat dichloride, I + pebulate, I + pendimethalin, I + penoxsulam, I + pentachlorophenol, I + pentanochlor, I + pentoxazone, I + pethoxamid, I + phenmedipham, I + picloram, I + picolinafen, I + pinoxaden, I + piperophos, I + pretilachlor, I + primisulfuron, I + primisulfuron-methyl, I + prodiamine, I + profoxydim, I + prohexadione-calcium, I + prometon, I + prometryn, I + propachlor, I + propanil, I + propaquizafop, I + propazine, I + propham, I + propisochlor, I + propoxycarbazone, I + propoxycarbazone-sodium, I + propyzamide, I + prosulfocarb, I + prosulfuron, I + pyraclonil, I + pyraflufen, I + pyraflufen-ethyl, I + pyrasulfotole, I + pyrazolynate, I + pyrazosulfuron, I + pyrazosulfuron-ethyl, I + pyrazoxyfen, I + pyribenzoxim, I + pyributicarb, I + pyridafol, I + pyridate, I + pyriftalid, I + pyriminobac, I + pyriminobac-methyl, I + pyrimisulfan, I + pyrithiobac, I + pyrithiobac-sodium, I + pyroxasulfone, I + pyroxsulam, I + quinclorac, I + quinmerac, I + quinoclamine, I + quizalofop, I + quizalofop-P, I + rimsulfuron, I + saflufenacil, I + sethoxydim, I + siduron, I + simazine, I + simetryn, I + sodium chlorate, I + sulcotrione, I + sulfentrazone, I + sulfometuron, I + sulfometuron-methyl, I + sulfosate, I + sulfosulfuron, I + sulfuric acid, I + tebuthiuron, I + tefuryltrione, I + tembotrione, I + tepraloxydim, I + terbacil, I + terbumeton, I + terbuthylazine, I + terbutryn, I + thenylchlor, I + thiazopyr, I + thifensulfuron, I + thiencarbazone, I + thifensulfuron-methyl, I + thiobencarb, I + topramezone, I + tralkoxydim, I + tri-allate, I + triasulfuron, I + triaziflam, I + tribenuron, I + tribenuron-methyl, I + triclopyr, I + trietazine, I + trifloxysulfuron, I + trifloxysulfuron-sodium, I + trifluralin, I + triflusulfuron, I + triflusulfuron-methyl, I + trihydroxytriazine, I + trinexapac-ethyl, I + tritosulfuron, I + [3-[2-chloro-4-fluoro-5-(1-methyl-6-trifluoromethyl-2,4-dioxo-1,2,3,4-tetrahydropyrimidin-3-yl)phenoxy]-2-pyridyloxy]acetic acid ethyl ester (CAS RN 353292-31-6). The compounds of the present invention may also be combined with herbicidal compounds disclosed in WO06/024820 and/or WO07/096576.

The mixing partners of the compound of Formula (I) may also be in the form of esters or salts, as mentioned e.g. in The Pesticide Manual, Sixteenth Edition, British Crop Protection Council, 2012.

The compound of Formula (I) can also be used in mixtures with other agrochemicals such as fungicides, nematicides or insecticides, examples of which are given in The Pesticide Manual.

The mixing ratio of the compound of Formula (I) to the mixing partner is preferably from 1: 100 to 1000:1.

The mixtures can advantageously be used in the above-mentioned formulations (in which case "active ingredient" relates to the respective mixture of compound of Formula (I) with the mixing partner).

The compounds of Formula (I) according to the invention can also be used in combination with one or more safeners. Likewise, mixtures of a compound of Formula (I) according to the invention with one or more further herbicides can also be used in combination with one or more safeners. The safeners can be AD 67 (MON 4660), benoxacor, cloquintocet-mexyl, cyprosulfamide (CAS RN 221667-31-8), dichlormid, fenchlorazole-ethyl, fenclorim, fluxofenim, furilazole and the corresponding R isomer, isoxadifen-ethyl, mefenpyr-diethyl, oxabetrinil, N-isopropyl-4-(2-methoxy-benzoylsulfamoyl)-benzamide (CAS RN 221668-34-4). Other possibilities include safener compounds disclosed in, for example, EP0365484 e.g N-(2-methoxybenzoyl)-4-[(methylaminocarbonyl)amino]benzenesulfonamide. Particularly preferred are mixtures of a compound of Formula (I) with cyprosulfamide, isoxadifen-ethyl, cloquintocet-mexyl and/or N-(2-methoxybenzoyl)-4-[(methylaminocarbonyl)amino]benzenesulfonamide.

The safeners of the compound of Formula (I) may also be in the form of esters or salts, as mentioned e.g. in The Pesticide Manual, 16th Edition (BCPC), 2012. The reference to cloquintocet-mexyl also applies to a lithium, sodium, potassium, calcium, magnesium, aluminium, iron, ammonium, quaternary ammonium, sulfonium or phosphonium salt thereof as disclosed in WO 02/34048, and the reference to fenchlorazole-ethyl also applies to fenchlorazole, etc.

Preferably the mixing ratio of compound of Formula (I) to safener is from 100:1 to 1:10, especially from 20:1 to 1:1.

The mixtures can advantageously be used in the above-mentioned formulations (in which case "active ingredient" relates to the respective mixture of compound of Formula (I) with the safener).

The present invention still further provides a method of controlling weeds at a locus comprising crop plants and weeds, wherein the method comprises application to the locus of a weed controlling amount of a composition according to the present invention. 'Controlling' means killing, reducing or retarding growth or preventing or reducing germination. Generally the plants to be controlled are unwanted plants (weeds). 'Locus' means the area in which the plants are growing or will grow.

The rates of application of compounds of Formula (I) may vary within wide limits and depend on the nature of the soil, the method of application (pre- or post-emergence; seed dressing; application to the seed furrow; no tillage application etc.), the crop plant, the weed(s) to be controlled, the prevailing climatic conditions, and other factors governed by the method of application, the time of application and the target crop. The compounds of Formula (I) according to the invention are generally applied at a rate of from 10 to 2000 g/ha, especially from 50 to 1000 g/ha.

The application is generally made by spraying the composition, typically by tractor mounted sprayer for large areas, but other methods such as dusting (for powders), drip or drench can also be used.

Useful plants in which the composition according to the invention can be used include crops such as cereals, for example barley and wheat, cotton, oilseed rape, sunflower, maize, rice, soybeans, sugar beet, sugar cane and turf.

Crop plants can also include trees, such as fruit trees, palm trees, coconut trees or other nuts. Also included are vines such as grapes, fruit bushes, fruit plants and vegetables.

Crops are to be understood as also including those crops which have been rendered tolerant to herbicides or classes of herbicides (e.g. ALS-, GS-, EPSPS-, PPO-, ACCase- and HPPD-inhibitors) by conventional methods of breeding or by genetic engineering. An example of a crop that has been rendered tolerant to imidazolinones, e.g. imazamox, by conventional methods of breeding is Clearfield® summer rape (canola). Examples of crops that have been rendered tolerant to herbicides by genetic engineering methods include e.g. glyphosate- and glufosinate-resistant maize varieties commercially available under the trade names RoundupReady® and LibertyLink®.

Crops are also to be understood as being those which have been rendered resistant to harmful insects by genetic engineering methods, for example Bt maize (resistant to European corn borer), Bt cotton (resistant to cotton boll weevil) and also Bt potatoes (resistant to Colorado beetle). Examples of Bt maize are the Bt 176 maize hybrids of NK® (Syngenta Seeds). The Bt toxin is a protein that is formed naturally by *Bacillus thuringiensis* soil bacteria. Examples of toxins, or transgenic plants able to synthesise such toxins, are described in EP-A-451 878, EP-A-374 753, WO 93/07278, WO 95/34656, WO 03/052073 and EP-A-427 529. Examples of transgenic plants comprising one or more genes that code for an insecticidal resistance and express one or more toxins are KnockOut® (maize), Yield Gard® (maize), NuCOTIN33B® (cotton), Bollgard® (cotton), NewLeaf® (potatoes), NatureGard® and Protexcta®. Plant crops or seed material thereof can be both resistant to herbicides and, at the same time, resistant to insect feeding ("stacked" transgenic events). For example, seed can have the ability to express an insecticidal Cry3 protein while at the same time being tolerant to glyphosate.

Crops are also to be understood to include those which are obtained by conventional methods of breeding or genetic engineering and contain so-called output traits (e.g. improved storage stability, higher nutritional value and improved flavour).

Other useful plants include turf grass for example in golf-courses, lawns, parks and roadsides, or grown commercially for sod, and ornamental plants such as flowers or bushes.

The compositions can be used to control unwanted plants (collectively, 'weeds'). The weeds to be controlled may be both monocotyledonous species, for example Agrostis, Alopecurus, Avena, Brachiaria, Bromus, Cenchrus, Cyperus, Digitaria, Echinochloa, Eleusine, Lolium, Monochoria, Rottboellia, Sagittaria, Scirpus, Setaria and Sorghum, and dicotyledonous species, for example Abutilon, Amaranthus, Ambrosia, Chenopodium, Chrysanthemum, Conyza, Galium, Ipomoea, Nasturtium, Sida, Sinapis, Solanum, Stellaria, Veronica, Viola and Xanthium. The compounds of the present invention have been shown to exhibit particularly good activity against certain grass weed species, especially *Lolium Perenne.* Weeds can also include plants which may be considered crop plants but which are growing outside a crop area ('escapes'), or which grow from seed left over from a previous planting of a different crop ('volunteers'). Such volunteers or escapes may be tolerant to certain other herbicides.

The compounds of the present invention can be prepared according to the following schemes.

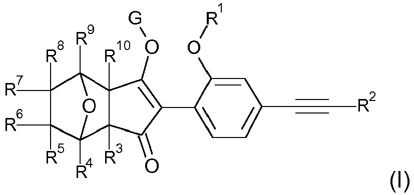

Compounds of Formula (I) wherein G is other than hydrogen may be prepared by treating a compound of formula (A), which is a compound of Formula (I) wherein G is hydrogen, with a reagent G-Z, wherein G-Z is an alkylating agent such as an alkyl halide, acylating agent such as an acid chloride or anhydride, sulfonylating agent such as a sulfonyl chloride, carbamylating agent such as a carbamoyl chloride, or carbonating agent such as a chloroformate, using known methods.

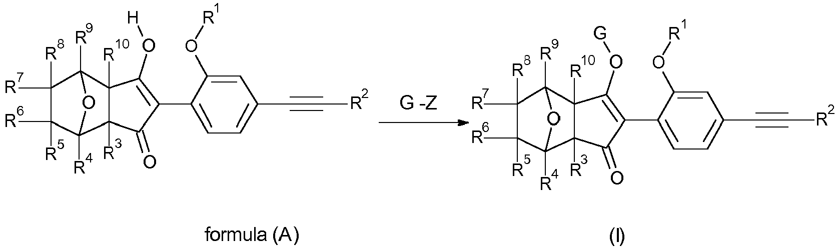

A compound of formula (A) may be prepared by the cyclisation of a compound of formula (B), wherein R is hydrogen or an alkyl group, preferably in the presence of an acid or base, and optionally in the presence of a suitable solvent, by analogous methods to those described by T. Wheeler, US4209532. The compounds of formula (B) have been particularly designed as intermediates in the synthesis of the compounds of the Formula (I). A compound of formula (B) wherein R is hydrogen may be cyclised under acidic conditions, preferably in the presence of a strong acid such as sulfuric acid, polyphosphoric acid or Eaton's reagent, optionally in the presence of a suitable solvent such as acetic acid, toluene or dichloromethane.

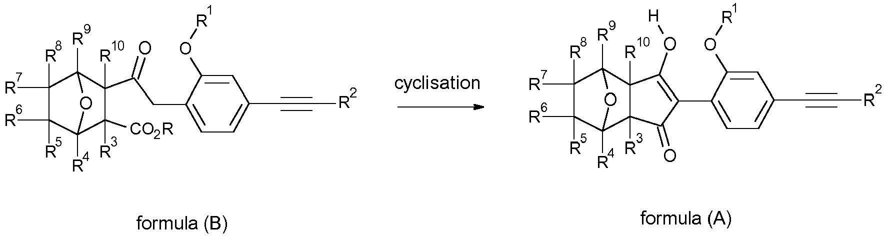

A compound of formula (B) wherein R is alkyl (preferably methyl or ethyl), may be cyclised under acidic or basic conditions, preferably in the presence of at least one equivalent of a strong base such as potassium *tert*-butoxide, lithium diisopropylamide or sodium hydride and in a solvent such as tetrahydrofuran, toluene, dimethylsulfoxide or *N*,*N*-dimethylformamide.

A compound of formula (B), wherein R is hydrogen, may be prepared by saponification of a compound of formula (C) wherein R' is alkyl (preferably methyl or ethyl), under standard conditions, followed by acidification of the reaction mixture to effect decarboxylation, by similar processes to those described, for example, by T. Wheeler, US4209532.

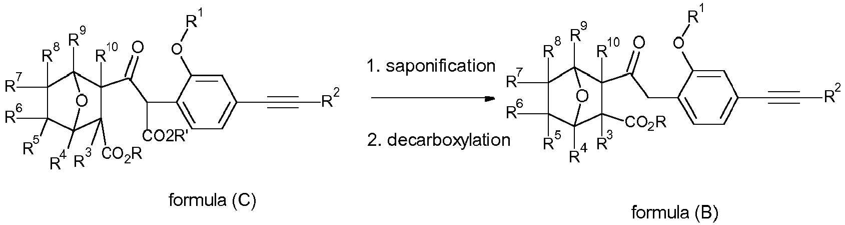

A compound of formula (B), wherein R is hydrogen, may be esterified to a compound of formula (B), wherein R is alkyl, under standard conditions, for example by heating with an alkyl alcohol, ROH, in the presence of an acid catalyst.

A compound of formula (C), wherein R and R' is alkyl, may be prepared by treating a compound of formula (D) with a suitable carboxylic acid chloride of formula (E) under basic conditions. Suitable bases include potassium *tert*-butoxide, sodium bis(trimethylsilyl)amide and lithium diisopropylamide and the reaction is preferably conducted in a suitable solvent (such as tetrahydrofuran or toluene) at a temperature of between -80 °C and 30 °C. Alternatively, a compound of formula (C), wherein R is H, may be prepared by treating a compound of formula (D) with a suitable base (such as potassium *tert*-butoxide, sodium bis(trimethylsilyl)amide and lithium diisopropylamide) in a suitable solvent (such as tetrahydrofuran or toluene) at a suitable temperature (between -80 °C and 30 °C) and reacting the resulting anion with a suitable anhydride of formula (F):

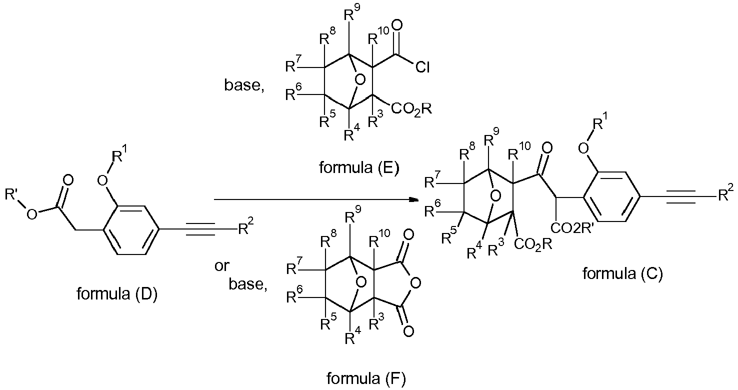

Compounds of formula (D) are known compounds, or may be prepared from known compounds by known methods.

A compound of formula (E) may be prepared from a compound of formula (F) by treatment with an alkyl alcohol, R-OH, in the presence of a base, such as dimethylaminopyridine or an alkaline metal alkoxide (see, for example, S. Buser and A. Vasella, Helv. Chim. Acta, (2005), 88, 3151, M. Hart et al., Bioorg. Med. Chem. Letters, (2004), 14, 1969), followed by treatment of the resulting acid with a chlorinating reagent such as oxalyl chloride or thionyl chloride under known conditions (see, for example, C. Santelli-Rouvier. Tetrahedron Lett., (1984), 25 (39), 4371; D. Walba and M. Wand, Tetrahedron Lett., (1982), 23 (48), 4995; J. Cason, Org. Synth. Coll. Vol. III, (169), 1955).

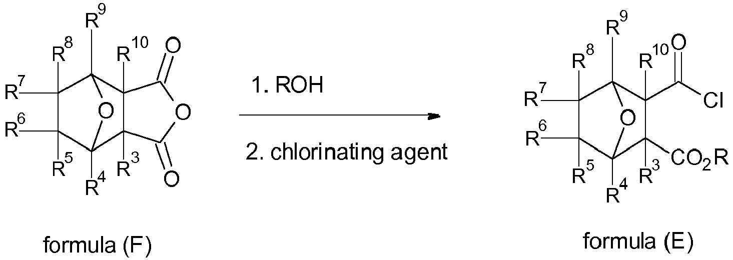

A compound of formula (F) wherein R⁵ and R⁸ are hydrogen may be prepared by the reduction of a compound of formula (G) under known conditions (see, for example, Y. Baba, N. Hirukawa and M. Sodeoka, Bioorg. Med. Chem. (2005), 13 (17), 5164, M. Hart et al., Bioorg. Med. Chem. Letters, (2004), 14 (18), 1969, Y. Baba, N. Hirukawa, N. Tanohira and M. Sodeoka, J. Am. Chem. Soc., (2003), 125, 9740).

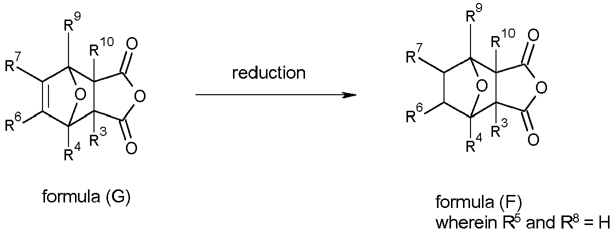

A compound of formula (G) may be prepared by reacting a compound of formula (H) with an anhydride of formula (J), optionally in the presence of a Lewis acid catalyst using known procedures.

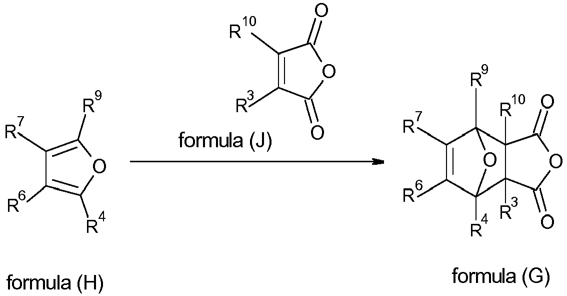

Compounds of formula (H) and formula (J) are known compounds, or may be made from known compounds by known methods.

Compounds of formula (G) are alkenes, and as such undergo further reactions typical of alkenes to give additional compounds of formula (F) according to known procedures. Examples of such reactions include, but are not restricted to, halogenation, epoxidation, cyclopropanation, dihydroxylation, hydroarylation, hydrovinylation and hydration of alkenes. In turn, the products from these reactions may be transformed into additional compounds of formula (F) by methods described, for example by J. March, Advanced Organic Chemistry, third edition, John Wiley and Sons. Compounds of formula (G) wherein R⁶ or R⁷ are C₁-C₆alkoxy are enol ethers, and these may be hydrolysed to the corresponding ketone using standard procedures to give additional compounds of formula (F). Certain compounds of formula (F), for example where R⁵ is a halogen, may be converted into compounds of formula (G) by known methods.

A compound of formula (G) may also be prepared by reacting a compound of formula (H) with a compound of formula (K), wherein R" is hydrogen or an alkyl group, to give a compound of formula (L) and cyclising a compound of formula (L) under known conditions (see, for example, P. Sprague et al., J. Med. Chem., (1985), 28, 1580, A. Guzaev and M. Manoharan, J. Am. Chem. Soc., (2003), 125, 2380, and A. Marchand and R. Allen, J. Org. Chem., (1975), 40 (17), 2551.

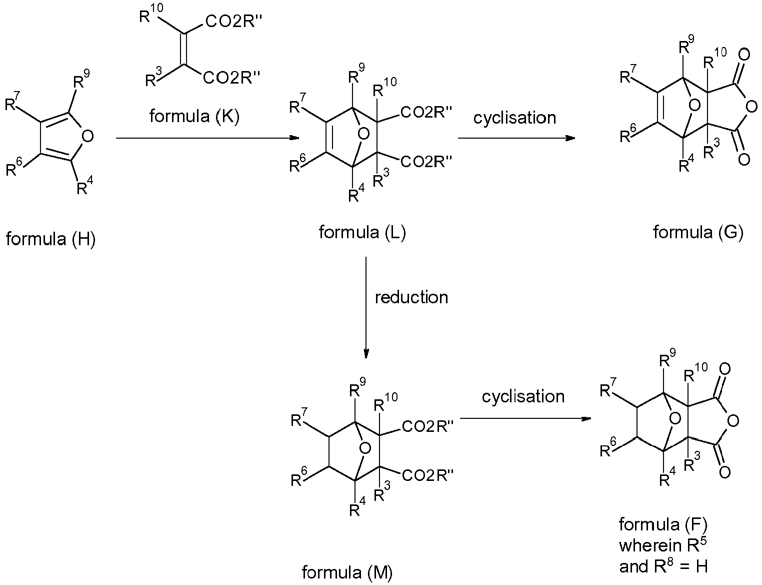

A compound of formula (L) may also be reduced to a compound of formula (M), and a compound of formula (M) cyclised to a compound of formula (F) wherein R⁵ and R⁸ are hydrogen, under conditions similar to those described previously.

Compounds of formula (K) are known compounds, or may be prepared from known compounds by known methods.

Additional compounds of formula (A) may be prepared by reacting an iodonium ylide of formula (N), wherein Ar is an optionally substituted phenyl group, and an aryl boronic acid of formula (O), in the presence of a suitable palladium catalyst, a base and in a suitable solvent.

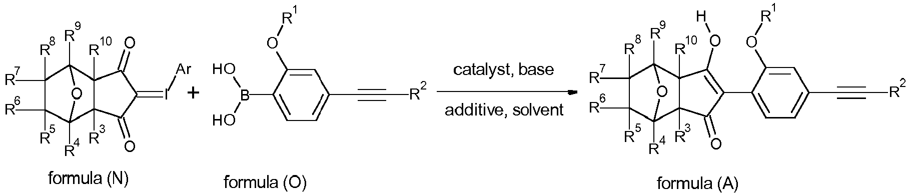

Suitable palladium catalysts are generally palladium(II) or palladium(0) complexes, for example palladium(II) dihalides, palladium(II) acetate, palladium(II) sulfate, bis(triphenylphosphine)-palladium(II) dichloride, bis(tricyclopentylphosphine)-palladium(II) dichloride, bis(tricyclohexyl-phosphine)palladium(II) dichloride, bis(dibenzylideneacetone)palladium(0) or tetrakis-(triphenylphosphine)palladium(0). The palladium catalyst can also be prepared *"in situ"* from palladium(II) or palladium(0) compounds by complexing with the desired ligands, by, for example, combining the palladium(II) salt to be complexed, for example palladium(II) dichloride (PdCl₂) or palladium(II) acetate (Pd(OAc)₂), together with the desired ligand, for example triphenylphosphine (PPh₃), tricyclopentylphosphine, tricyclohexylphosphine, 2-dicyclohexylphosphino-2',6'-dimethoxybiphenyl or 2-dicyclohexylphosphino-2',4',6'-triisopropylbiphenyl and the selected solvent, with a compound of formula (N), the arylboronic acid of formula (O), and a base. Also suitable are bidendate ligands, for example 1,1'-bis(diphenylphosphino)ferrocene or 1,2-bis(diphenylphosphino)ethane. By heating the reaction medium, the palladium(II) complex or palladium(0) complex desired for the C-C coupling reaction is thus formed *"in situ",* and then initiates the C-C coupling reaction.

The palladium catalysts are used in an amount of from 0.001 to 50 mol %, preferably in an amount of from 0.1 to 15 mol %, based on the compound of formula (N). The reaction may also be carried out in the presence of other additives, such as tetralkylammonium salts, for example, tetrabutylammonium bromide. Preferably the palladium catalyst is palladium acetate, the base is lithium hydroxide and the solvent is aqueous 1,2-dimethoxyethane.

A compound of formula (N) may be prepared from a compound of formula (P) by treatment with a hypervalent iodine reagent such as a (diacetoxy)iodobenzene or an iodosylbenzene and a base such as aqueous sodium carbonate, lithium hydroxide or sodium hydroxide in a solvent such as water or an aqueous alcohol such as aqueous ethanol according to the procedures of K. Schank and C. Lick, Synthesis, (1983), 392, R. M. Moriarty et al., J. Am. Chem. Soc, (1985), 107, 1375, or of Z. Yang et al., Org. Lett., (2002), 4 (19), 3333.

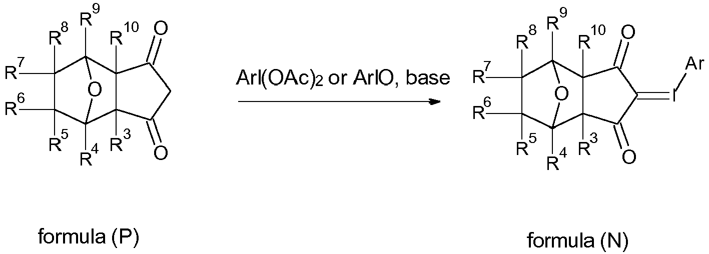

A compound of formula (P) wherein R⁵ and R⁸ are hydrogen may be prepared by reduction of a compound of formula (R) under known conditions.

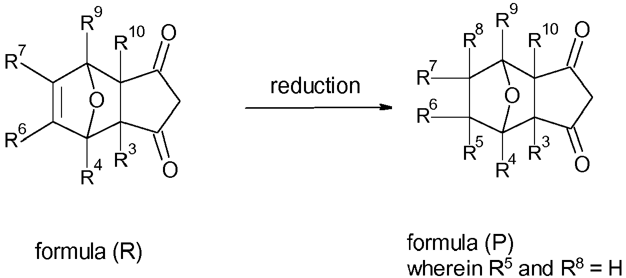

Compounds of formula (R) are alkenes, and as such undergo further reactions typical of alkenes to give additional compounds of formula (P) according to known procedures. Examples of such reactions include, but are not restricted to, halogenation, epoxidation, cyclopropanation, dihydroxylation, hydroarylation, hydrovinylation and hydration of alkenes. In turn, the products of these reactions may be transformed into additional compounds of formula (P) by methods described, for example by J. March, Advanced Organic Chemistry, third edition, John Wiley and Sons. Compounds of formula (R) wherein R⁶ or R⁷ are C₁-C₆alkoxy are enol ethers, and these may be hydrolysed to the corresponding ketone using standard procedures. In turn, the ketone may be further transformed, for example by ketalisation, oximation, reduction and the like under known conditions to give additional compounds of formula (P).

A compound of formula (R) may be prepared by reacting a compound of formula (H) with a cyclopentenedione of formula (T), optionally in the presence of a Lewis acid catalyst, according to procedures described, for example by B. Zwanenburg et al., Tetrahedron (1989), 45 (22), 7109 and by M. Oda et al., Chem. Lett., (1977), 307.

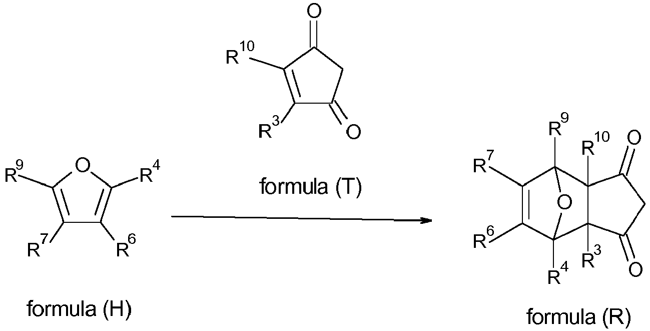

Compounds of formula (H) and formula (T) are known compounds or may be made from known compounds by known methods.

In a further approach, a compound of formula (A) may be prepared from a compound of Formula (I), wherein G is C₁-₄ alkyl, by hydrolysis, preferably in the presence of an acid catalyst such as hydrochloric acid and optionally in the presence of a suitable solvent such as tetrahydrofuran, acetone or 4-methylpentan-2-one.

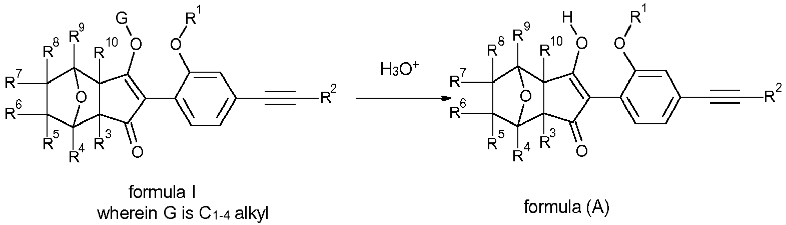

A compound of Formula (I) wherein G is C₁-₄ alkyl, may be prepared from a compound of formula (U), wherein G is C₁₋₄ alkyl, and Hal is a halogen (preferably bromine or iodine), by coupling with an aryl boronic acid of formula (O), in the presence of a suitable palladium catalyst and a base and preferably in the presence of a suitable ligand, and in a suitable solvent. Preferably the palladium catalyst is palladium acetate, the base is potassium phosphate, the ligand is 2-dicyclohexylphosphino-2',6'-dimethoxybiphenyl and the solvent is toluene.

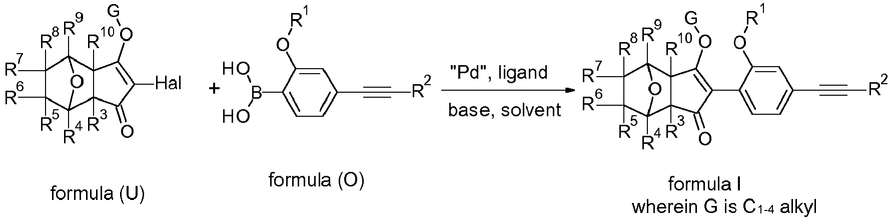

A compound of formula (U) may be prepared by halogenation of a compound of formula (P), followed by reaction of the resulting halide of formula (V) with a C₁₋₄ alkyl halide or tri-C₁₋₄-alkylorthoformate under known conditions (for example by the procedures of R. Shepherd and A. White, J. Chem. Soc. Perkin Trans. 1 (1987), 2153, and Y.-L. Lin et al., Bioorg. Med. Chem. (2002), 10, 685). Alternatively, a compound of formula (U) may be prepared by reaction of a compound of formula (P) with a C₁₋₄ alkyl halide or a tri-C₁₋₄-alkylorthoformate, and halogenation of the resulting enone of formula (W) under known conditions.

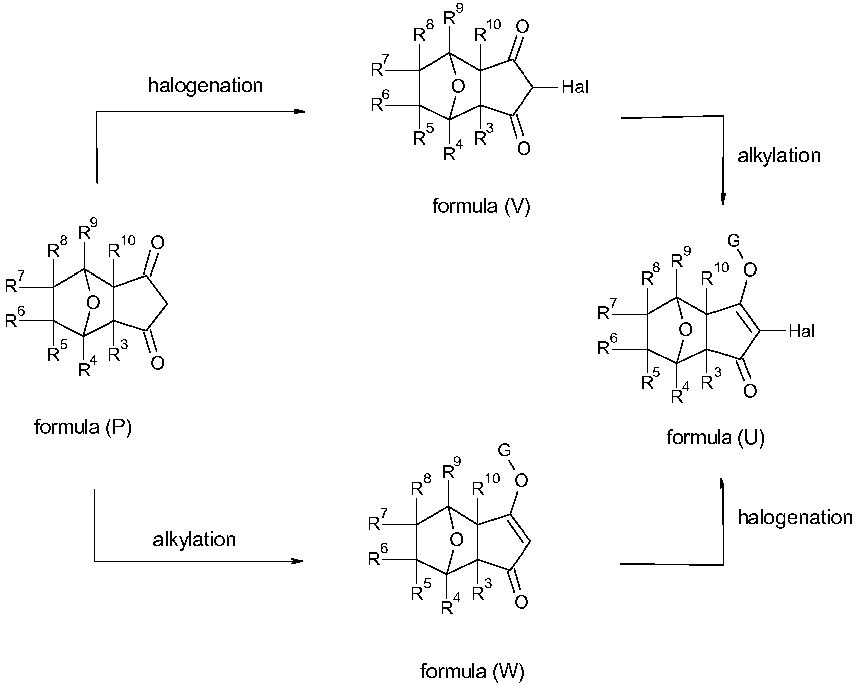

A compound of formula (O) may be prepared from an aryl halide of formula (X), wherein Hal is bromine or iodine, by known methods (see, for example, W. Thompson and J. Gaudino, J. Org. Chem, (1984), 49, 5237 and R. Hawkins et al., J. Am. Chem. Soc., (1960), 82, 3053). For example, an aryl halide of formula (X) may be treated with an alkyl lithium or alkyl magnesium halide in a suitable solvent, preferably diethyl ether or tetrahydrofuran, at a temperature of between -80 °C and 30 °C, and the aryl magnesium or aryl lithium reagent obtained may then be reacted with a trialkyl borate (preferably trimethylborate) to give an aryl dialkylboronate which may be hydrolysed to provide a boronic acid of formula (O) under acidic conditions. 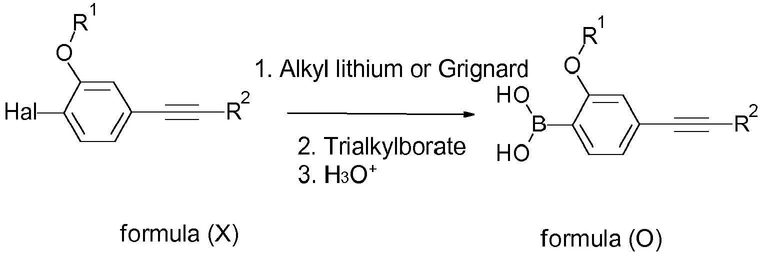 Alternatively a compound of formula (X) may be reacted with a cyclic boronate ester derived from a 1,2- or a 1,3-alkanediol such as pinacol, 2,2-dimethyl-1,3-propanediol and 2-methyl-2,4-pentanediol) under known conditions (see, for example, N. Miyaura et al., J. Org. Chem., (1995), 60, 7508, and W. Zhu and D. Ma, Org. Lett., (2006), 8 (2), 261), and the resulting boronate ester may be hydrolysed under acidic conditions to give a boronic acid of formula (O).

An aryl halide of formula (X) may be prepared from an aniline of formula (Y) by known methods, for example the Sandmeyer reaction, *via* the corresponding diazonium salts.

Anilines of formula (Y) are known compounds, or may be made from known compounds, by known methods.

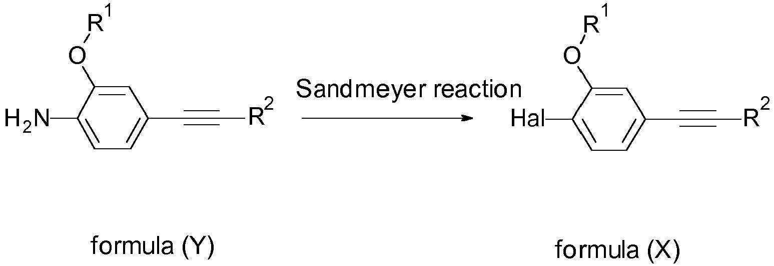

Additional compounds of formula (A) may be prepared by reacting a compound of formula (P), or a compound of formula (R), with an organolead reagent of formula (Z) under conditions described, for example, by J. Pinhey, Pure and Appl. Chem., (1996), 68 (4), 819 and by M. Moloney et al., Tetrahedron Lett., (2002), 43, 3407.

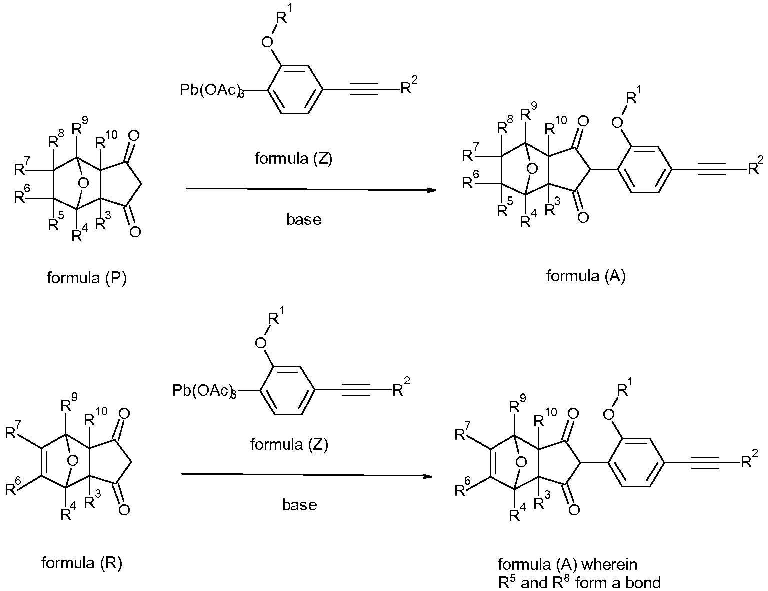

The organolead reagent of formula (Z) may be prepared from a boronic acid of formula (O), a stannane of formula (AA), wherein R is C₁-C₄ alkyl or by direct plumbation of a compound of formula (AB) with lead tetraacetate according to known procedures.

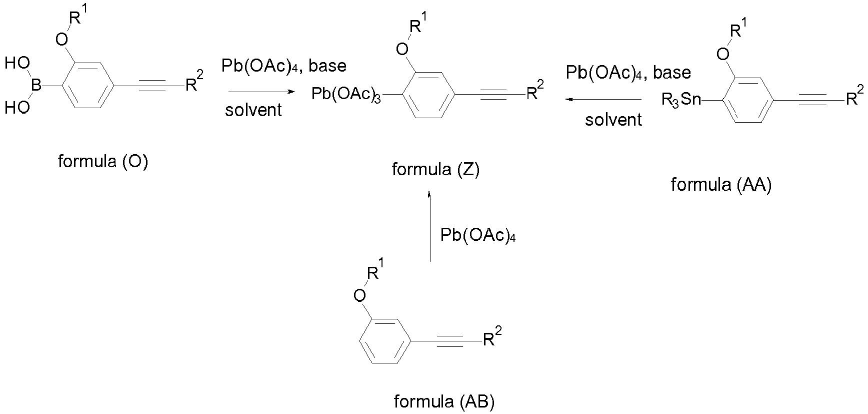

Further compounds of formula (A) may be prepared by reacting a compound of formula (P) or a compound of formula (R) with suitable triarylbismuth compound under conditions described, for example, by A. Yu. Fedorov et al., Russ. Chem. Bull. Int. Ed., (2005), 54 (11), 2602, and by P. Koech and M. Krische, J. Am. Chem. Soc., (2004), 126 (17), 5350 and references therein.

Compounds of formula (I) can be made form compounds of formula (BA), wherein LG is halogen or other suitable leaving group (such as an alkyl or arylsulfonate), similary to methods described in WO2014/191534A1.

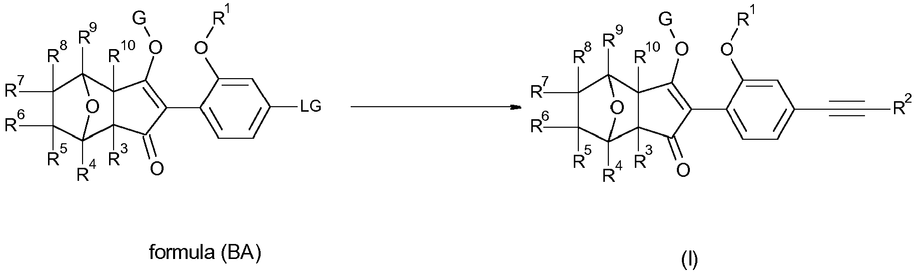

In a further approach, a compound of Formula (BA) may be prepared from a compound of formula (AC) by suitable derivatisation under standard conditions.

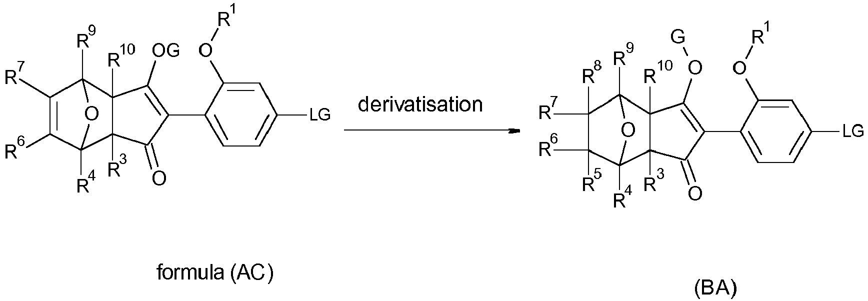

For example, compounds of formula (AC) are alkenes, and as such undergo further reactions typical of alkenes to give compounds of Formula (BA) according to known procedures. Examples of such reactions include, but are not restricted to, reduction, halogenation, epoxidation, cyclopropanation, dihydroxylation, hydroarylation, hydrovinylation and hydration. Compounds of formula (AC) wherein R⁶ or R⁷ is bromine or iodine are vinyl halides, and undergo known reactions of vinyl halides such as Suzuki-Miyaura, Sonogashira, Stille and related reactions. Certain other compounds of formula (AC), wherein R⁶ or R⁷ is C₁-C₆alkoxy, are enol ethers, and these may be hydrolysed to the corresponding ketone using standard procedures. In turn, the ketone produced may be further transformed, for example by ketalisation, oximation, reduction and the like under known conditions to give additional compounds of Formula (BA). Similarly, compounds of formula (AC) wherein R⁶ or R⁷ is C₁-C₆amino or di-C₁-C₆amino are enamines, and these also may be hydrolysed to the corresponding ketone using standard procedures.

A compound of formula (AC), wherein G is C₁-C₄ alkyl, may be prepared from a compound of formula (AD), wherein G is C₁-C₄ alkyl and X is halogen or other suitable leaving group (such as an alkyl or arylsulfonate, or an arylselenoxide), by reaction with a compound of formula (H), optionally in a suitable solvent, and optionally in the presence of a suitable base.

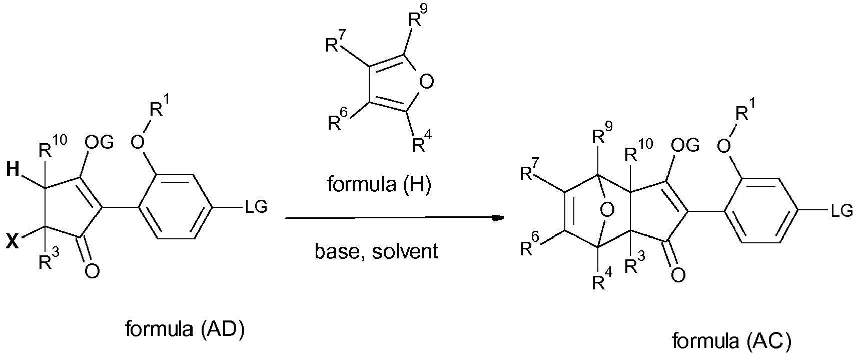

Suitable solvents include toluene, dichloromethane and chloroform and suitable bases include organic bases such as triethylamine, Hunig's base and 1,8-diazabicyclo[5.4.0]undec-7-ene. Preferably the solvent is toluene and the base is 1,8-diazabicyclo[5.4.0]undec-7-ene.

A compound of formula (AD) may be prepared from a compound of formula (AE), under known conditions.

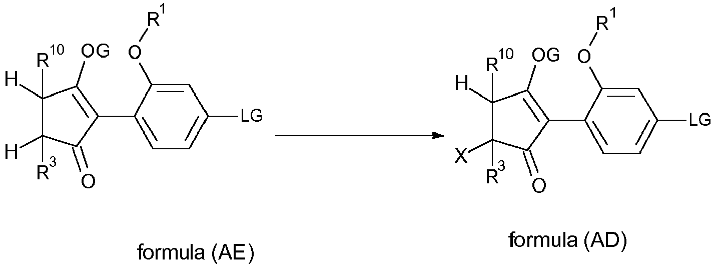

For example, a compound of formula (AD) wherein X is chlorine may be prepared by reacting a compound of formula (AE) with copper(II) chloride and lithium chloride according to the procedure of E. Kosower et al., J. Org. Chem., (1963), 28, 630.

Compounds of formula (AE) are known compounds or may be made from known compounds by known methods (see, for example, Y. Song, B. Kim and J-N Heo, Tetrahedron Lett., (2005), 46, 5977). Alternatively, a compound of formula (AE) wherein G is C₁-C₄alkyl may be prepared from a compound of formula (AE), wherein G is hydrogen, for example by reaction with a C₁₋₄ alkyl halide or a tri-C₁₋₄-alkylorthoformate. Compounds of formula (AE), wherein G is hydrogen, are known, or may be prepared from known compounds by known methods (see, for example, T. Wheeler, US4338122, US4283348, J. T. Kuethe et al., J. Org. Chem., (2002), 67, 5993, S. Buchwald et al., J. Am. Chem. Soc., (2003), 125, 11818).

Alternatively, a compound of formula (AE), wherein G is C₁₋₄alkyl, may be prepared by reacting a compound of formula (AF), wherein G is C₁₋₄alkyl and Z is a halogen, preferably bromine or iodine, with a boronic acid of formula (BB) in the presence of a suitable metal catalyst, a suitable base, and optionally a suitable ligand, in a suitable solvent.

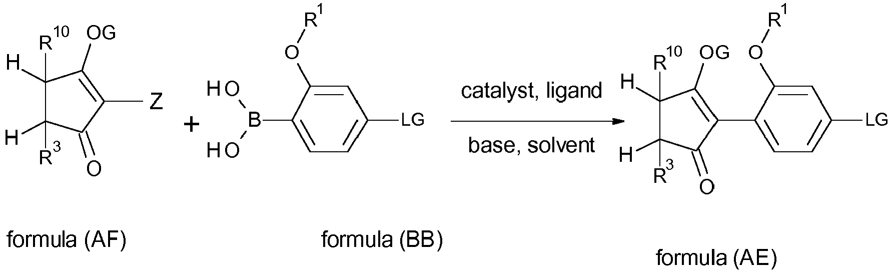

Suitable solvents include toluene and n-butanol, suitable bases include inorganic bases such as potassium phosphate, a suitable metal catalyst is a palladium catalyst, for example in the form of palladium(II) acetate, and suitable ligands include substituted phosphines, for example 2-dicyclohexylphosphino-2',6'-dimethoxybiphenyl.

Compounds of formula (AF) are known compounds, or may be prepared by methods known in the literature. For example a compound of formula (AF) wherein G is C₁₋₄alkyl and Z is a bromine atom may be prepared by reacting a compound of formula (AG), wherein G is C₁₋₄alkyl, with a suitable brominating agent, such as N-bromosuccinimide, in a suitable solvent, such as 1,2-dichloroethane, as described by R. Shepherd and A. White, J. Chem. Soc. Perkin Trans. 1 (1987), 10, 2153.

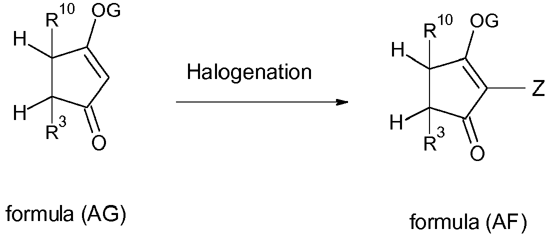

In a similar manner, a compound of formula (BC) may be prepared from a compound of formula (AH) by suitable derivatisation under standard conditions.

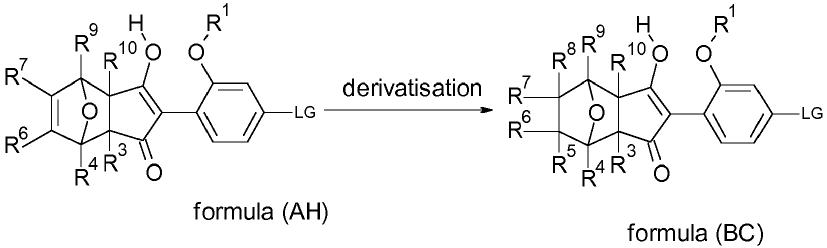

For example, compounds of formula (AH) are alkenes, and as such undergo further reactions typical of alkenes to give compounds of formula (BC) according to known procedures. Examples of such reactions include, but are not restricted to, reduction, halogenation, epoxidation, cyclopropanation, dihydroxylation, hydroarylation, hydrovinylation and hydration. Compounds of formula (AH) wherein R⁶ or R⁷ is bromine or iodine are vinyl halides, and undergo known reactions of vinyl halides such as Suzuki-Miyaura, Sonogashira, Stille and related reactions. Certain other compounds of formula (AH), wherein R⁶ or R⁷ is C₁-C₆alkoxy, are enol ethers, and these may be hydrolysed to the corresponding ketone using standard procedures. In turn, the ketone produced may be further transformed, for example by ketalisation, oximation, reduction and the like under known conditions to give additional compounds of formula (BC). Similarly, compounds of formula (AH) wherein R⁶ or R⁷ is C₁-C₆amino or di-C₁-C₆amino are enamines, and these also may be hydrolysed to the corresponding ketone using standard procedures.

A compound of formula (AH) may be prepared from a compound of formula (Al) by reaction with a compound of formula (H), optionally in a suitable solvent, and optionally in the presence of a suitable catalyst. The compounds of formula (Al) have been particularly designed as intermediates in the synthesis of the compounds of the Formula (I).

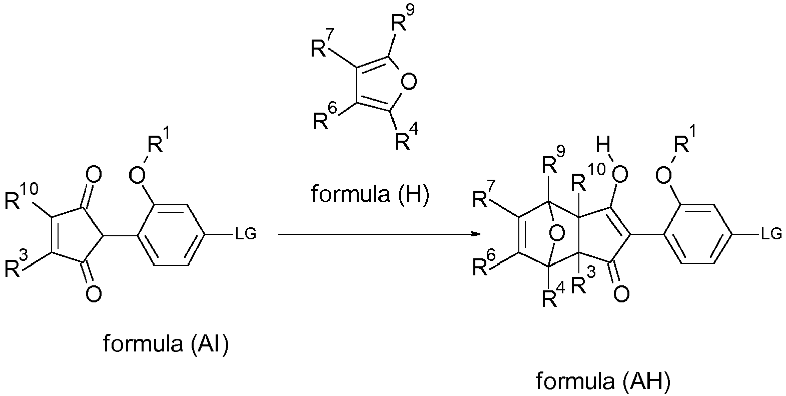

Preferably the catalyst is a Lewis acid catalyst such as aluminium chloride, bismuth (III) chloride, bismuth (III) trifluoromethanesulfonate, boron trifluoride, cerium (III) chloride, copper (I) trifluoromethanesulfonate, diethylaluminium chloride, hafnium (IV) chloride, iron (III) chloride, lithium perchlorate, lithium trifluoromethanesulfonate, magnesium bromide, magnesium iodide, scandium (III) trifluoromethanesulfonate, tin (IV) chloride, titanium (IV) chloride, titanium (IV) isopropoxide, trimethyl aluminium, *N*-trimethylsilyl-bis(trifluoromethanesulfonyl)imide, trimethylsilyl trifluoromethanesulfonate, ytterbium (III) trifluoromethanesulfonate, zinc iodide and zirconium (IV) chloride. Magnesium iodide is particularly preferred. Suitable solvents include those which are known to be effective solvents for conducting Diels-Alder reactions, among them, for example, chloroform, dichloromethane, diethyl ether, ethanol, methanol, perfluorinated alkanes, such as perfluorohexane, toluene, water, and ionic liquids such as 1-butyl-3-methylimidazolium tetrafluoroborate and 1-butyl-3-methylimidazolium hexafluorophosphate. Dichloromethane is particularly preferred as a solvent.

A compound of formula (Al), may be prepared by oxidising a compound of formula (AJ) in a suitable solvent such as toluene, acetone, chloroform, dichloromethane or 1,4-dioxane. A wide range of oxidants are suitable for effecting this transformation, including inorganic oxidants such as chromium trioxide, pyridinium dichromate, manganese dioxide and aluminium alkoxides such as aluminium isopropoxide, as well as organic oxidants such as 2,3-dichloro-5,6-dicyano-p-benzoquinone and hypervalent iodine oxidants such as 1,1,1,-tris(acetyloxy)-1,1-dihydro-1,2-benziodoxol-3-(1H)-one (Dess-Martin periodinane), Suitable procedures are described, for example, by K. Saito and H. Yamachika, US4371711. and by G. Piancatelli et al., Tetrahedron (1978), 34, 2775. The use of chromium trioxide in a mixture of sulfuric acid and acetone (Jones reagent) is preferred.

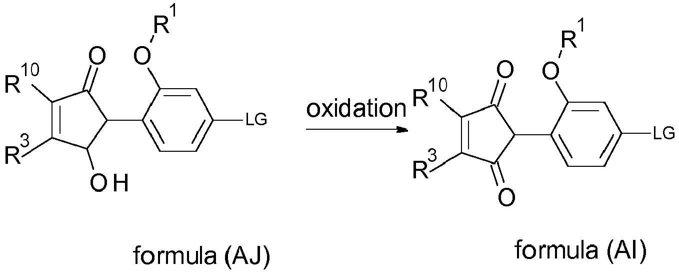

The compounds of the formula AI have been particularly designed as intermediates for the synthesis of the compounds of the Formula (I).

Particularly useful compounds of the formula AI are those, wherein R³ and R¹⁰ are hydrogen.

A compound of formula (AJ) may be prepared from a compound of formula (AK) by treatment with a suitable acid catalyst optionally in the presence of water and optionally in the presence of a suitable solvent, according to known procedures.

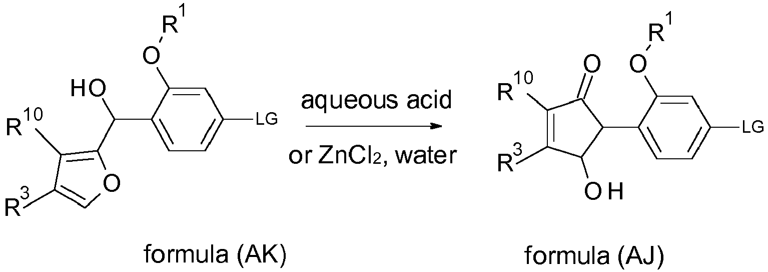

For example, a compound of formula (AK) may be converted to a compound of formula (AJ) in the presence of an aqueous solution of an acid such as phosphoric acid or polyphosphoric acid as described, for example by K. Saito and H. Yamachika, US4371711. Alternatively a compound of formula (AJ) may be prepared from a compound of formula (AK) by rearrangement in the presence of a Lewis acid catalyst such as zinc chloride according to the procedure of G. Piancatelli et al., Tetrahedron, (1978), 34, 2775.

A compound of formula (AK) may be prepared by the reduction of a compound of formula (AL) by known conditions (see, for example R Silvestri et al., J. Med. Chem., 2005, 48, 4378-4388).

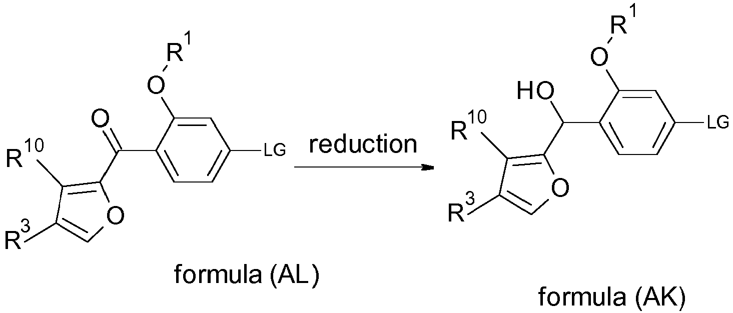

Compounds of formula (AL) are known, or may be made by known methods from known compounds (see, for example, L. Liebeskind et al., Org. Lett., (2003), 5 (17), 3033-3035, H. Firouzabadi, N. Iranpoor and F. Nowrouzi, Tetrahedron, (2004), 60,10843, R. Silvestri et al., J. Med. Chem., (2005), 48, 4378 and references therein).

Alternatively a compound of formula (AK) may be prepared by the addition of a suitable organometallic reagent such as an arylmagnesium halide of formula (AM) wherein Hal is a halide such as chloride, bromide or iodide, or an aryllithium reagent of formula (AN) or a diarylzinc reagent of formula (AO) to a furan-2-carboxaldehyde of formula (AP) according to known procedures (see, for example G. Panda et al., Tetrahedron Lett., (2005), 46, 3097).

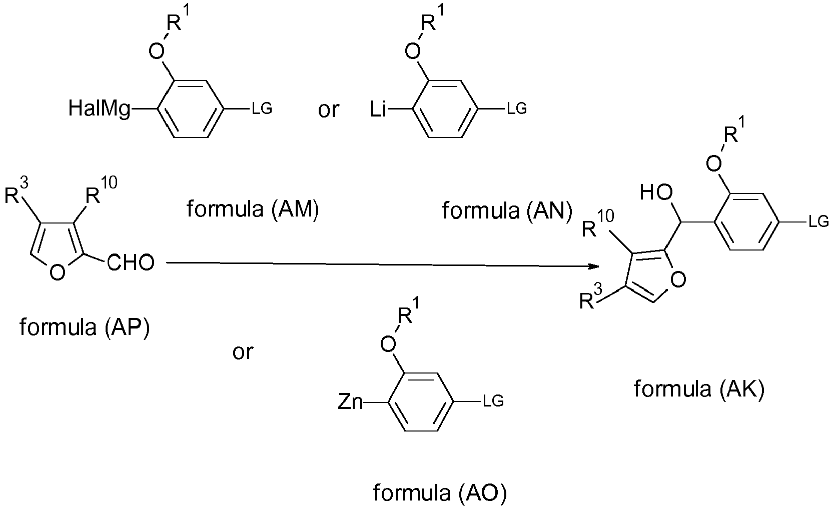

Additionally, compounds of formula (AK) may be prepared from compounds of formula (AR) by reaction with a strong base, for a example an alkyl lithium reagent such as *n*-butyllithium, optionally in the presence of an additive such as tetramethylethylenediamine, and in a suitable solvent such as diethyl ether or tetrahydrofuran, followed by reaction with a benzaldehyde of formula (AS) as described, for example by I. Gupta and M. Ravikanth, J. Org. Chem., (2004), 69, 6796, A. M. Echavarren et al., J. Am. Chem. Soc., (2003),125 (19), 5757, and by T. K. Chandrashekar et al., J. Org. Chem., (2002), 67, 6309-6319.

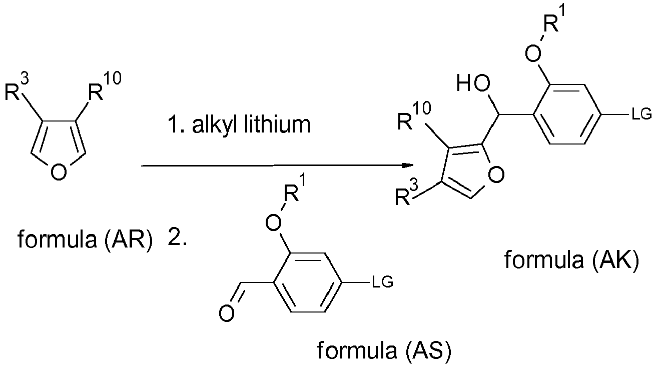

The organometallic reagents of formula (AM), formula (AN) and formula (AO) are known compounds or may be made by known methods from known compounds. Compounds of formula (AP), formula (AR) and formula (AS) are known compounds, or may be prepared from known compounds by known methods.

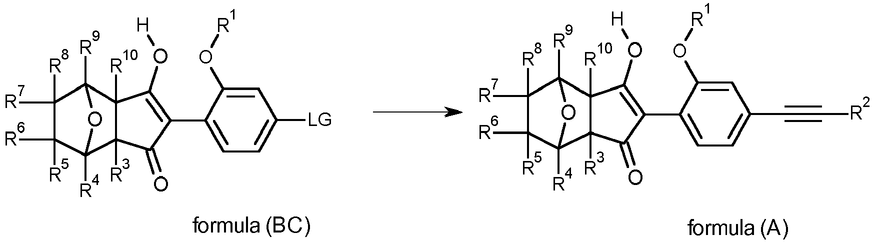

Compounds of formula (A) can be made form compounds of formula (BC), wherein LG is halogen or other suitable leaving group (such as an alkyl or arylsulfonate), similary to methods described in WO2014/191534A1.

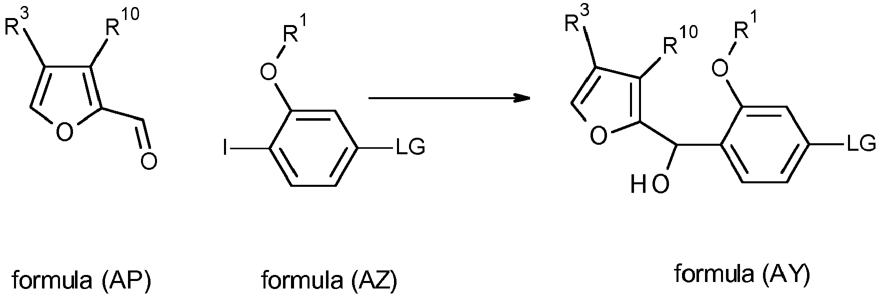

Compounds of formula (AK) can be prepared from compounds of formula (AP) and (AZ), wherein LG is halogen or other suitable leaving group (such as an alkyl or arylsulfonate), similarly to compounds of formula (AK) as previously discussed.

The following non-limiting examples provide specific synthesis methods for representative compounds of the present invention, as referred to in Table 1 below.

### Example 1: Synthesis of 4-[2-methoxy-4-(prop-1-yn-1-yl)phenyl]-10-oxatricyclo[5.2.1.0^{2,6}]decane-3,5-dione (Compound 1.2)

### Step 1: Synthesis of (4-bromo-2-methoxy-phenyl)-(2-furyl)methanol

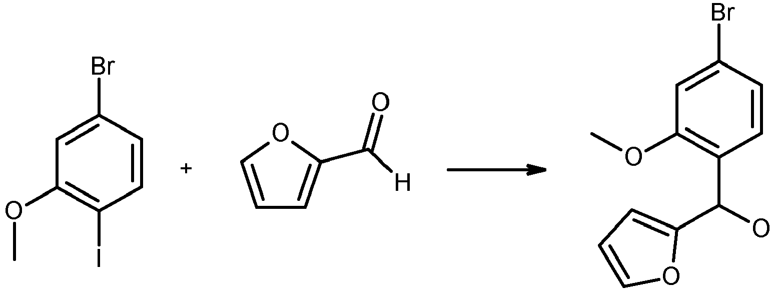

To a solution of 4-bromo-1-iodo-2-methoxy-benzene (11.44 mmol, 3.581 g) in dry THF (17.91 mL) under an atmosphere of nitrogen at -78°C was added a solution of isopropylmagnesium chloride in THF (1.3 mol/L, 11.0 mL, 14.30 mmol) over a period of 10 minutes, maintaining the reaction temperature between -65 and -70°C. After stirring for 20 minutes, the reaction mixture was warmed to room temperature over one hour. The reaction mixture was cooled again to -78°C and a solution of furan-2-carbaldehyde (13.73 mmol, 1.319 g) in THF (3.581 mL) was added over 5 minutes, whilst maintaining the reaction temperature between -65 and -70°C. After 30 minutes, the reaction was allowed to warm to room temperature and stirred for a further 60 minutes. The reaction was quenched by the addition of saturated aqueous ammonium chloride. The aqueous layer was extracted with ethyl acetate, the combined organic phase was dried over magnesium sulfate and the solvent removed *in vacuo.* The resulting residue was purified by silica gel flash chromatography (gradient elution: 0-30% ethyl acetate in hexane) to produce (4-bromo-2-methoxyphenyl)-(2-furyl)methanol as an orange oil (1.55g, 48%). ¹H NMR (400MHz, CDCl₃) 7.42 - 7.34 (m, 1H), 7.26 (s, 1H), 7.17 - 7.09 (m, 1H), 7.06 - 6.98 (m, 1H), 6.39 - 6.28 (m, 1H), 6.13 - 6.06 (m, 1H), 6.04 - 5.89 (m, 1H), 3.82 (s, 3H), 2.91 - 2.60 (m, 1H)

### Step 2: Synthesis of 5-(4-bromo-2-methoxy-phenyl)-4-hydroxy-cyclopent-2-en-1-one

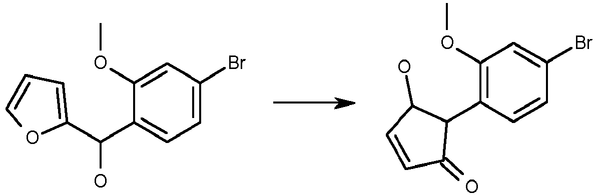

(4-bromo-2-methoxy-phenyl)-(2-furyl)methanol (5.485 mmol, 1.55 g) was dissolved in acetone (31 mL) and water (6 mL) and heated to 55°C. Phosphoric acid (0.796 mmol, 0.078 g, 0.039 mL) was added to the mixture and heated to 65°C and stirred overnight (16 h). The reaction mixture was cooled to room temperature and the acetone was removed *in vacuo.* The resulting mixture was diluted with water and extracted with ethyl acetate. The combined organic phase was dried over magnesium sulphate and concentrated *in vacuo.* The resulting residue was purified by silica gel flash chromatography (gradient elution: 0-80% ethyl acetate in hexane) to produce 5-(4-bromo-2-methoxy-phenyl)-4-hydroxy-cyclopent-2-en-1-one as a brown oil (0.968 g). ¹H NMR (400MHz, CDCl₃) 7.58 - 7.46 (m, 1H), 7.16 - 7.06 (m, 1H), 7.02 (d, 2H), 6.42 - 6.23 (m, 1H), 5.06 - 4.76 (m, 1H), 3.74 (s, 3H), 3.47 - 3.32 (m, 1H), 2.45 - 2.26 (m, 1H)

### Step 3: Synthesis of 2-(4-bromo-2-methoxy-phenyl)cyclopent-4-ene-1,3-dione

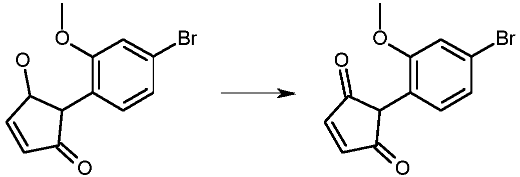

Concentrated sulfuric acid (3.94 mL) and trioxochromium (3.83 mmol, 0.383 g) was added to water (19.7 mL) at 0°C to generate Jones' reagent. 5-(4-bromo-2-methoxyphenyl)-4-hydroxy-cyclopent-2-en-1-one (3.48 mmol, 0.986 g) was dissolved in acetone (12.8 mL), cooled to 0°C and treated with the Jones' reagent. After 20 minutes, the mixture was warmed to room temperature and stirred for 1h 40. The reaction was quenched with the addition of *iso*-propanol (20 mL) and allowed to stir for a further 2 h. The acetone was removed *in vacuo* and the residue was partitioned between water and ethyl acetate. The aqueous layer was extracted with ethyl acetate (3 x 30 mL), the combined organic layers were washed with water (2 x 30 mL) and brine (30 mL), then dried over magnesium sulfate. The solution was concentrated *in vacuo* and the resulting residue was purified by silica gel flash chromatography (gradient elution: 0-100% ethyl acetate in hexane) to produce 2-(4-bromo-2-methoxy-phenyl)cyclopent-4-ene-1,3-dione as a yellow oil (0.480 g). ¹H NMR (400MHz, CDCl₃) 7.33 (s, 2H), 7.18 - 7.07 (m, 1H), 7.03 (s, 1H), 6.97 - 6.92 (m, 1H), 3.82 (s, 1H), 3.64 (s, 3H)

### Step 4: Synthesis of 4-(4-bromo-2-methoxyphenyl)-10-oxatricyclo[5.2.1.0^{2,6}]dec-8-ene-3,5-dione

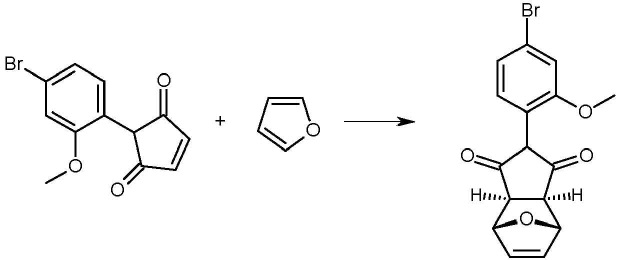

The 2-(4-bromo-2-methoxy-phenyl)cyclopent-4-ene-1,3-dione (1.71 mmol, 0.480 g) was suspended in dichloromethane (59.9 mmol, 5.09 g, 3.84 mL), and furan (5.12 mmol, 0.349 g, 0.373 mL) and diiodomagnesium (0.342 mmol, 0.0950 g) were added. The mixture was stirred in the dark for 1 week over which time a dark orange solid formed. Methanol was added to the mixture and stirred to dissolve the solid residue, then the solution was concentrated *in vacuo* and the resulting residue was purified by silica gel flash chromatography (gradient elution: 0-10% methanol in DCM) to produce 4-(4-bromo-2-methoxyphenyl)-10-oxatricyclo[5.2.1.0^{2,6}]dec-8-ene-3,5-dione (0.319 g) as a yellow foam. ¹H NMR (400MHz, CD₃OD) 7.19 - 6.95 (m, 3H), 6.53 (t, 2H), 4.95 (t, 2H), 3.76 (s, 3H), 2.87 - 2.67 (m, 2H)

### Step 5: Synthesis of 4-(4-bromo-2-methoxyphenyl)-10-oxatricyclo[5.2.1.0^{2,6}]decane-3,5-dione

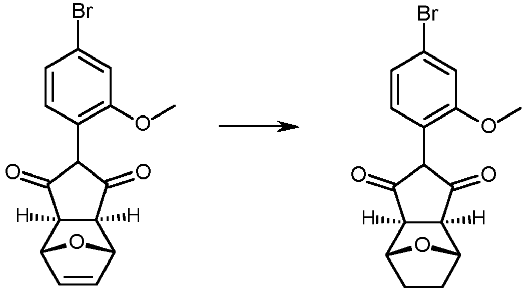

4-(4-bromo-2-methoxyphenyl)-10-oxatricyclo[5.2.1.0^{2,6}]dec-8-ene-3,5-dione (0.853 mmol, 0.298 g) was dissolved in methanol (6 mL) and ethyl acetate (2 mL), and charged with a slurry of 1% platinum on carbon in methanol (3 mL). The mixture was stirred under 1.5 bar pressure of hydrogen for 2 hours. The reaction mixture was filtered through celite (HiFlo™) and washed with methanol. The filtrate was concentrated in vacuo to produce 4-(4-bromo-2-methoxyphenyl)-10-oxatricyclo[5.2.1 .0^{2,6}]decane-3,5-dione (0.280 g) as a yellow solid. ¹H NMR (400MHz, CD₃OD) 7.19 - 6.90 (m, 3H), 4.67 - 4.51 (m, 2H), 3.76 (s, 3H), 3.34 (s, 1H), 2.81 (s, 2H), 1.88 - 1.74 (m, 2H), 1.70 - 1.54 (m, 2H)

### Step 6: Synthesis of 4-[2-methoxy-4-(prop-1-yn-1-yl)phenyl]-10-oxatricyclo[5.2.1.0^{2,6}]decane-3,5-dione

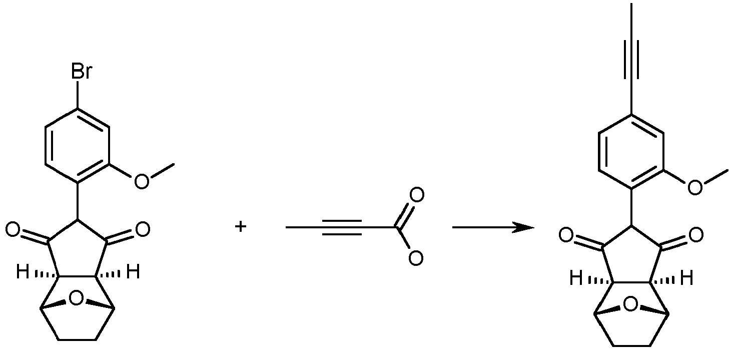

4-(4-bromo-2-methoxyphenyl)-10-oxatricyclo[5.2.1.0^{2,6}]decane-3,5-dione (0.769 mmol, 0.270 g), but-2-ynoic acid (0.923 mmol, 0.0776 g), dichlorobis(triphenylphosphine)palladium(II) (0.0384 mmol, 0.0273 g), and 1,4-bis-(diphenylphosphino)butane (0.0769 mmol, 0.0328 g) were suspended in DMSO (9.23 mL). After the addition of DBU (2.31 mmol, 0.351 g, 0.344 mL), the reaction mixture was stirred at 110°C for 45 minutes in the microwave. The reaction mixture was poured into an aqueous solution of potassium carbonate and extracted with ethyl acetate. The organic layer was discarded, and the aqueous layer was acidified and re-extracted with ethyl acetate. The combined organic layers were dried over magnesium sulfate and concentrated *in vacuo.* The resulting residue was purified by silica gel flash chromatography (gradient elution: 0-10% methanol in dichloromethane) to produce 4-[2-methoxy-4-(prop-1-yn-1-yl)phenyl]-10-oxatricyclo[5.2.1.0^{2,6}]decane-3,5-dione (0.164g) as a white solid. ¹H NMR (400MHz, CD₃OD) 7.10 - 7.01 (m, 1H), 6.93 (s, 2H), 4.63 - 4.52 (m, 2H), 3.73 (s, 3H), 2.72 (s, 2H), 2.01 (s, 3H), 1.85 - 1.72 (m, 2H), 1.67 - 1.55 (m, 2H)

### Example 2: Synthesis of racemic isobutyric acid 4-(2-methoxy-4-prop-1-ynyl-phenyl)-5-oxo-10-oxa-tricyclo[5.2.1.0^{2,6}]dec-3-en-3-yl ester (Compound 1.1)

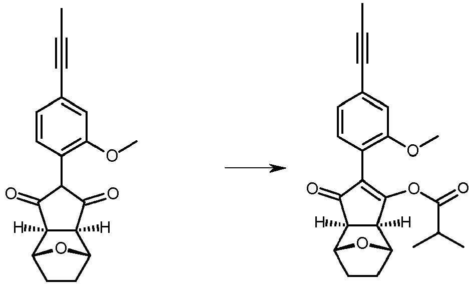

4-[2-methoxy-4-(prop-1-yn-1-yl)phenyl]-10-oxatricyclo [5.2.1.0^{2,6}]decane-3,5-dione (0.30 mmol, 0.087 g) was suspended in dichloromethane (4 mL) and treated with 2-methylpropanoyl chloride (0.35 mmol, 0.038 g) and N,N-diethylethanamine (0.35 mmol, 0.036 g, 0.049 mL). After 30 minutes, the reaction mixture was concentrated *in vacuo* and purified by silica gel flash chromatography (gradient elution: 0-60% ethyl acetate in hexane) to produce racemic isobutyric acid 4-(2-methoxy-4-prop-1-ynyl-phenyl)-5-oxo-10-oxa-tricyclo[5.2.1.0^{2,6}]dec-3-en-3-yl ester (89 mg) as white solid. ¹H NMR (400MHz, CDCl₃) δ = 7.32 - 7.10 (m, 2H), 7.03 - 6.82 (m, 1H), 4.73 (d, 1H), 4.55 (d, 1H), 3.46 (d, 1H), 2.76 (d, 1H), 2.69 - 2.52 (m, 1H), 2.13 (s, 3H), 2.04 (s, 3H), 1.96 - 1.76 (m, 2H), 1.70 - 1.49 (m, 2H), 1.19 - 1.05 (m, 6H).

### Example 3: Synthesis of racemic 8-ethyl-4-[2-methoxy-4-(prop-1-yn-1-yl)phenyl]-10-oxatricyclo[5.2.1.0^{2,6}]decane-3,5-dione (Compound 1.8).

### Step 1: Synthesis of racemic 4-(4-bromo-2-methoxyphenyl)-8-ethenyl-10-oxatricyclo[5.2.1.0^{2,6}]-decane-3,5-dione

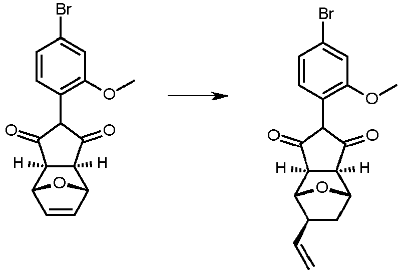

4-(4-bromo-2-methoxyphenyl)-10-oxatricyclo[5.2.1.0^{2,6}]dec-8-ene-3,5-dione (1.289 mmol, 450 mg) was dissolved in DMF (13 mL) and tributyl(pentyl)ammonium chloride (376 mg, 1.289 mmol), sodium formic acid (267 mg, 3.866 mmol) and iodoethylene (1.289 mmol) were added. The reaction mixture was heated under microwave irridation at 150 °C for 25 mins. The Reaction mixture was poured on 2M aqueous HCI, and extracted with ethyl acetate. The combined organics were concentrated *in vacuo* and purified by silica gel flash chromatography (gradient elution: 0-10% methanol in ethyl acetate) to produce racemic 4-(4-bromo-2-methoxyphenyl)-8-ethenyl-10-oxatricyclo[5.2.1.0^{2,6}]-decane-3,5-dione (230 mg, 0.340 mmol, 26%). ¹H NMR (400 MHz, CD₃OD) 7.12 - 7.14 (1 H, m), 7.07 - 7.11 (1 H, m), 7.02 - 7.04 (1 H, m), 5.76 - 7.01 (1 H, m), 5.06 (1 H, dd), 4.97 (1 H, dd), 4.61 (1 H, d), 4.30 (1 H, s), 3.75 (3 H, s), 2.87 (1 H, d), 2.82 (1 H, d), 2.58 (1 H, td), 1.96 (1 H, dd), 1.60 (1 H, dt).

### Step 2: Synthesis of racemic 4-(4-bromo-2-methoxyphenyl)-8-ethyl-10-oxatricyclo[5.2.1.0^{2,6}]-decane-3,5-dione

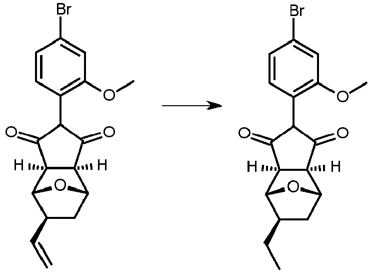

1% platinum on carbon (130 mg, paste with H₂O) was placed in a hydrogenation vessel. A solution of racemic 4-(4-bromo-2-methoxyphenyl)-8-ethenyl-10-oxatricyclo[5.2.1.0^{2,6}]-decane-3,5-dione (130 mg, 0.345 mmol) dissolved in methanol (4 mL) was added slowly to the above vessel followed by ethyl acetate (2 mL). The reaction vessel was sealed and stirred under a hydrogen atmosphere (1.5 bar) for 4 hours. The reaction mixture was was filtered through celite and the filtrate was concentrated *in vacuo* to afford racemic 4-(4-bromo-2-methoxyphenyl)-8-ethyl-10-oxatricyclo[5.2.1.0^{2,6}]-decane-3,5-dione (244 mg, 0.315 mmol, 91%). ¹H NMR (400MHz, CDCl₃) 7.20 (s, 2H), 4.55 (d, 1H), 4.29 (s, 1H), 2.87 - 2.81 (m, 2H), 2.06 (s, 6H), 1.93 - 1.78 (m, 2H), 1.53 - 1.46 (m, 1H), 1.43 - 1.35 (m, 1H), 1.32 - 1.25 (m, 1H), 1.00 -0.92 (m, 3H).

### Step 3: Synthesis of racemic 8-ethyl-4-[2-methoxy-4-(prop-1-yn-1-yl)phenyl]-10-oxatricyclo[5.2.1.0^{2,6}]decane-3,5-dione

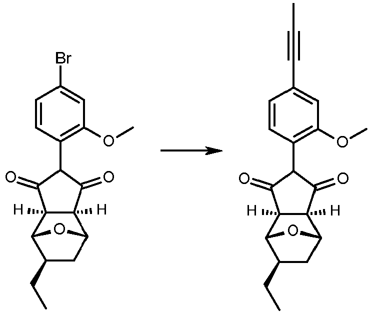

Racemic 4-(4-bromo-2-methoxyphenyl)-8-ethyl-10-oxatricyclo[5.2.1.0^{2,6}]-decane-3,5-dione (122 mg, 0.3217 mmol), 1,4-bis-(diphenylphosphino)butane (0.03217 mmol), dichlorobis(triphenylphosphine)palladium(II) (0.01609 mmol) and but-2-ynoic acid (0.3861 mmol) were placed into a microwave vial. DMSO (3.9 mL) and DBU (0.144 mL, 0.9652 mmol) were added to the vail and the reaction mixture heated under microwave irridation at at 110 °C for 45 mins. Further portions of but-2-ynoic acid (0.3861 mmol), 1,4-bis-(diphenylphosphino)butane (0.03217 mmol) and dichlorobis(triphenylphosphine)palladium(II) (0.01609 mmol) were added to the reaction mixture and heated under microwave irridation at 110 °C for a further 75 mins. The reaction was diluted with aqueous 2M HCI and extracted with ethyl acetateEthyl acetate. The organic layer were dried and concentrated *in vacuo* and purified by silica gel flash chromatography (gradient elution: 25-100% ethyl acetate in hexane) to produce Ethyl acetate racemic 8-ethyl-4-[2-methoxy-4-(prop-1-yn-1-yl)phenyl]-10-oxatricyclo[5.2.1.0^{2,6}]decane-3,5-dione (88 mg, 0.2600 mmol, 81%). ¹H NMR (400 MHz, CDCl₃) 8.56 - 9.51 (1 H, m), 7.71 - 7.79 (1 H, m), 7.13 (1 H, dd), 7.01 (1 H, d), 4.67 (1 H, d), 4.40 (1 H, s), 3.85 - 3.96 (3 H, m), 2.54 - 2.95 (2 H, m), 2.01 - 2.09 (3 H, m), 1.75 (2 H, s), 1.36 - 1.56 (2 H, m), 1.18 - 1.34 (1 H, m), 0.91 (3 H, t).

### Example 4: Synthesis of racemic 8-methoxymethyl-4-[2-methoxy-4-(prop-1-yn-1-yl)phenyl]-10-oxatricyclo[5.2.1.0^{2,6}]decane-3,5-dione.

### Step 1: Synthesis of racemic 4-(4-bromo-2-methoxyphenyl)-8-methoxymethyl-10-oxatricyclo[5.2.1.0^{2,6}]dec-8-ene-3,5-dione

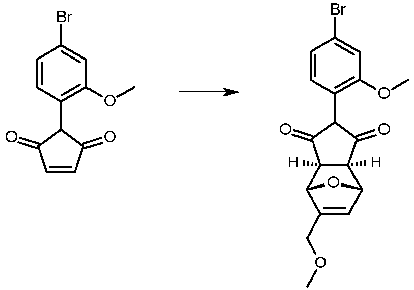

3-(methoxymethyl)furan (39.3 mmol), 2-(4-bromo-2-methoxy-phenyl)cyclopent-4-ene-1,3-dione (2.21 g, 7.86 mmol) and magnesium diiodide (1.28 mmol) suspended in dichloromethane (8 mL, 124.8 mmol) was stirred at room temperature for 18 days in the dark. The reaction mixture was concentrated *in vacuo* and purified by silica gel flash chromatography (gradient elution: 0-5% methanol in dichloromethane) to furnish a residue that was further purified by trituration from diethyl ether to give racemic 4-(4-bromo-2-methoxyphenyl)-8-methoxymethyl-10-oxatricyclo[5.2.1.0^{2,6}] dec-8-ene-3,5-dione (452 mg, 1.150 mmol, 15%). ¹H NMR (400 MHz, CD₃OD) 2.79 - 2.86 (m, 2 H) 3.36 (s, 3 H) 3.76 (s, 3 H) 4.88 - 4.89 (m, 1 H) 4.93 - 4.94 (m, 1 H) 6.29 - 6.34 (m, 1 H) 6.99 - 7.16 (m, 3 H).

### Step 2: Synthesis of racemic 4-(4-bromo-2-methoxyphenyl)-8-methoxymethyl-10-oxatricyclo[5.2.1.0^{2,6}]-decane-3,5-dione

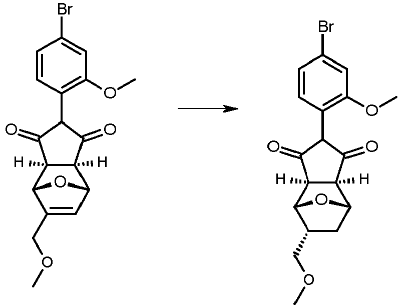

*N,N*-Diethylethanamine (3.449 mmol) was added to a solution of racemic 4-(4-bromo-2-methoxyphenyl)-8-methoxymethyl-10-oxatricyclo[5.2.1.0^{2,6}]dec-8-ene-3,5-dione (452 mg, 1.150 mmol) and 2,4,6-triisopropylbenzenesulfonohydrazide (3.449 mmol) in tetrahydrofuran (13 mL). The reaction mixture was then heated under reflux for 1 hour. The reaction mixture was cooled to room temperature, concentrated *in vacuo* and purified by silica gel flash chromatography (gradient elution: 5-100% ethyl acetate in *iso*-hexane) to give racemic 4-(4-bromo-2-methoxyphenyl)-8-methoxymethyl-10-oxatricyclo[5.2.1.0^{2,6}]-decane-3,5-dione (329 mg, 0.8325 mmol, 72%). ¹H NMR (400 MHz, CD₃OD) 1.24 - 1.28 (m, 1 H) 2.01 - 2.05 (m, 1 H) 2.42 - 2.53 (m, 1 H) 2.75 - 2.81 (m, 1 H) 3.05 - 3.09 (m, 1 H) 3.37 - 3.38 (m, 3H) 3.41 - 3.47 (m, 1 H) 3.54 - 3.59 (m, 1 H) 3.75 - 3.77 (m, 3 H) 4.52 - 4.57 (m, 2 H) 6.99 - 7.16 (m, 3 H)

### Step 3: Synthesis of racemic 8-methoxymethyl-4-[2-methoxy-4-(prop-1-yn-1-yl)phenyl]-10-oxatricyclo[5.2.1.0^{2,6}]decane-3,5-dione

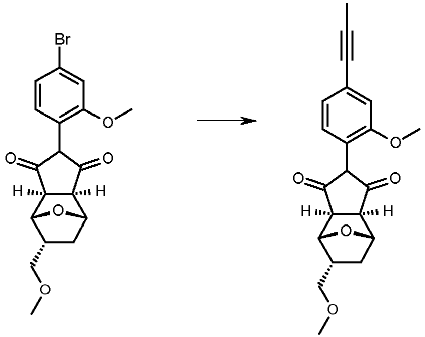

Racemic 4-(4-bromo-2-methoxyphenyl)-8-methoxymethyl-10-oxatricyclo[5.2.1.0^{2,6}]-decane-3,5-dione (329 mg, 0.8325 mmol), 1,4-bis-(diphenylphosphino)butane (0.08325 mmol), dichlorobis(triphenylphosphine)palladium(II) (0.04162 mmol) and but-2-ynoic acid (0.9990 mmol) were suspended in dimehtylsulfoxide (10 mL) and DBU (0.373 mL, 2.497 mmol). The reaction mixture was stirred under microwave irridiation at 110°C for 45 minutes. The mixture was diluted with 2M aqueous HCI and extracted with ethyl acetate. The organic layers were dried (MgSO₄), concentrated *in vacuo* and purified by silica gel flash chromatography (gradient elution: 5-100% ethyl acetate in iso-hexane) to give racemic 8-methoxymethyl-4-[2-methoxy-4-(prop-1-yn-1-yl)phenyl]-10-oxatricyclo[5.2.1.0^{2,6}]decane-3,5-dione (210 mg, 0.5926 mmol, 71%). ¹H NMR: (400 MHz, CD₃OD) 1.21 - 1.26 (m, 1 H) 1.99 - 2.08 (m, 4 H) 2.46 - 2.55 (m, 2 H) 2.78 - 2.82 (m, 1 H) 3.06 - 3.11 (m, 1 H) 3.37 - 3.41 (m, 3H) 3.43 - 3.50 (m, 1 H) 3.56 - 3.61 (m, 1 H) 3.74 - 3.79 (m, 3 H) 4.54 - 4.59 (m, 2 H) 6.96 - 7.09 (m, 3 H)

### Example 5: Chiral resolution to prepare enantiopure 8-methoxymethyl-4-[2-methoxy-4-(prop-1-yn-1-yl)phenyl]-10-oxatricyclo[5.2.1.0^{2,6}]decane-3,5-dione (Compounds 1.19 and 1.20)

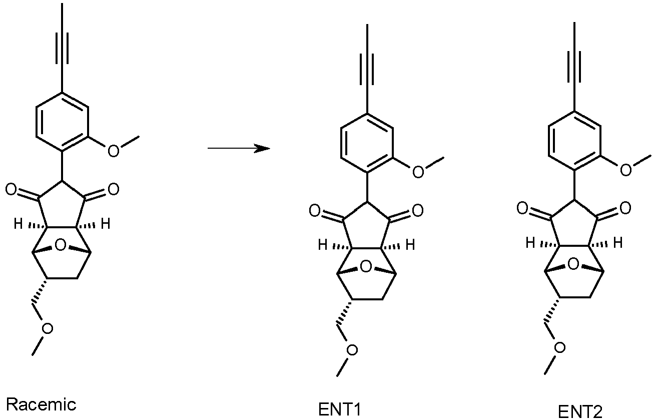

Racemic 8-methoxymethyl-4-[2-methoxy-4-(prop-1-yn-1-yl)phenyl]-10-oxatricyclo-[5.2.1.0^{2,6}]decane-3,5-dione (Compound 1.9) was separated into the enantiomer compounds 1.19 and 1.20 using a chiral HPLC column, by the following method and under the following conditions. The chiral HPLC column used was a (s,s) WhelkO1 - 5 micron - 21mm id x 250mm HPLC column, manufactured by Regis Technologies Inc. In this column, the chiral stationary phase is (S,S) 1-(3-5-dinitrobenzamido)-1,2,3,4-tetrahydrophenanthrene.

The solvent system used as an eluent for the column was a 40:60 (by volume) mixture of Solvent A and Solvent B, in which:
Solvent A is isohexane containing 0.1% isopropanol v/v and 0.15% acetic acid v/v, and
Solvent B is 80% isopropanol and 20% methanol
Other conditions were as follows:
   Flow rate through column: 21 mL/minute.
   Loading (compound loaded onto column): 5mg/ml in 1:1 isopropanol:methanol.
   Volume of sample (compound) injected per run = 0.90 to 0.95 mL
   Number of injections of compound = 24
   Length of run = 28 minutes
   Chiral HPLC on a total of 100 mg of the racemate under the above conditions gave 28.5 mg of 89.7% enantiomeric excess (e.e.) compound 1.19 at retention time 16.604 and 33.1 mg of 95.6% e.e compound 1.20 at retention time 19.581.

### Examples of herbicidal compounds of the present invention.

**Table 1**

| CMP | Structure | Chiral Info | NMR |
|---|---|---|---|
| 1.1 | 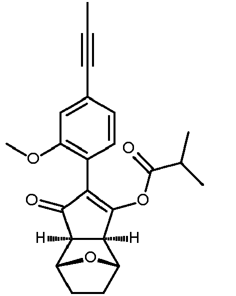 | Racemic | 1HNMR (400 MHz, CDCl₃) 7.21-7.11 (m, 1H), 7.05-6.95 (m, 1H), 6.95-6.86 (m , 1H), 4.80-4.69 (m, 1H), 4.59-4.48 (m, 1H), 3.72 (s, 3H), 3.57-3.42 (m, 1H), 2.85-2.74 (m, 1H), 2.73-2.57 (m, 1H), 2.05 (s, 3H), 1.93-1.75 (m, 2H), 1.66-1.57 (m, 2H), 1.19 (dd, 6H) |
| 1.2 | 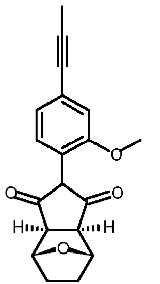 | Achiral | 1H NMR (400 MHz, CD₃OD) 7.10-7.01 (m, 1H), 6.93 (s, 2H), 4.63-4.52 (m, 2H), 3.73 (s, 3H), 2.8 (s, 2H), 2.01 (s, 3H), 1.85-1.72 (m, 2H), 1.67-1.55 (m, 2H) |
| 1.3 | 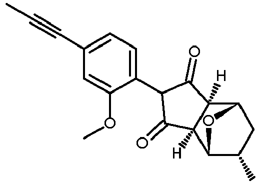 | Racemic | 1H NMR (400 MHz, CD₃OD) 7.08-7.04 (m, 1H), 6.97-6.92 (m, 2H), 4.54-4.51 (m, 1H), 4.39-4.36 (m, 1H), 3.74 (s, 3H), 3.13-3.07 (m, 1H), 2.83-2.79 (m, 1H), 2.32-2.24 (m, 1H), 2.15-2.06 (m, 1H), 2.02 (s, 3H), 1.14 (d, 3H), 1.09-1.05 (m, 1H) |
| 1.4 | 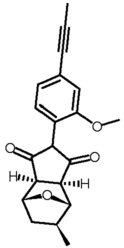 | Racemic | 1H NMR (400 MHz, CDCl₃) 9.15-9.04 (m, 1H), 7.80-7.73 (m, 1H), 7.18-7.11 (m, 1H), 7.05-7.00 (m, 1H), 4.72-4.65 (m, 1H), 4.32-4.25 (m, 1H), 3.92 (s, 3H), 2.88-2.79 (m, 1H), 2.73-2.63 (m, 1H), 2.06 (s, 3H), 2.01-1.90 (m, 1H), 1.88-1.78 (m, 1H), 1.44-1.28 (m, 1H), 1.09-0.99 (m, 3H) |
| 1.5 | 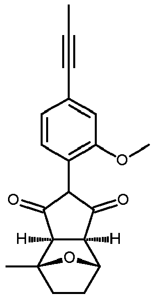 | Racemic | 1H NMR (400 MHz, CDCl₃) 7.76 (d, 1H), 7.14 (dd, 1H), 7.01 (d, 1H), 4.66 (d, 1H), 3.93 (s, 3H), 3.01-2.78 (m, 1H), 2.78-2.54 (m, 1H), 2.06 (s, 3H), 2.02-1.88 (m, 1H,), 1.76-1.60 (m, 3H), 1.58 (s, 3H,) |
| 1.6 | 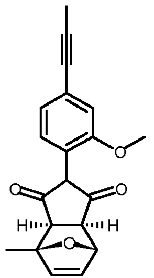 | Racemic | 1H NMR (400 MHz, CDCl₃) 9.27-8.94 (brs, 1H), 7.82-7.73 (m, 1H), 7.14 (dd, 1H), 7.02 (d, 1H), 6.49 (d, 1H), 6.31 (d, 1H), 5.03 (s, 1H), 3.96-3.92 (m, 3H), 3.03-2.79 (m, 1H), 2.78-2.46 (m, 1H), 2.06 (s, 3H), 1.70 (s, 3H) |
| 1.7 | 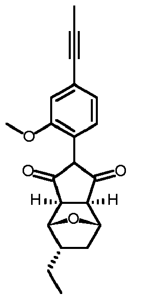 | Racemic | 1H NMR (400 MHz, CDCI3) 9.43-9.12 (brs, 1H), 7.79-7.72 (m, 1H), 7.13 (d, 1H), 7.01 (s, 1H), 4.69-4.56 (m, 2H), 3.92 (s, 3H), 3.27-2.92 (m, 1H), 2.82-2.63 (m, 1H), 2.18-2.06 (m, 1H), 2.03 (s, 3H), 1.80-1.71 (m, 1H), 1.55-1.36 (m, 2H), 1.31-1.22 (m, 1H), 0.99 (t, 3H) |
| 1.8 | 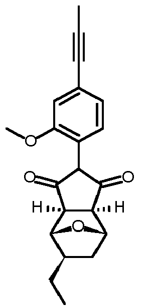 | Racemic | 1H NMR (400 MHz, CDCl₃) 9.51-8.56 (m, 1H), 7.79-7.71 (m, 1 H), 7.13 (dd, 1H), 7.01 (s, 1H), 4.67 (d, 1H), 4.40 (s, 1H), 3.96-3.85 (m, 3H,), 2.95-2.54 (m, 2H), 2.09-2.01 (m, 3H), 1.67-1.80 (m, 2H), 1.56-1.36 (m, 2H), 1.34-1.18 (m, 1H), 0.91 (t, 3H) |
| 1.9 | 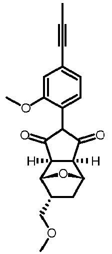 | Racemic | 1HNMR (400 MHz, CD₃OD) 7.09-6.96 (m, 3H), 4.59-4.54 (m, 2H), 3.79-3.74 (s, 3H), 3.61-3.56 (m, 1H), 3.50-3.43 (s, 1H), 3.41-3.37 (m, 3H), 3.11-3.06 (m, 1H), 2.82-2.78 (m, 1H), 2.55-2.46 (m, 2H), 2.08-1.99 (m, 4H), 1.21-1.26 (m, 1H) |
| 1.10 | 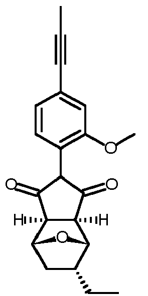 | Racemic | 1H NMR (400 MHz, CD₃OD) 7.07-7.03 (m, 1H), 6.93-6.89 (m, 2H), 4.54-4.50 (m, 1H), 4.47-4.44 (m, 1H), 3.72 (s, 3H), 2.96-2.92 (m, 1H), 2.67-2.64 (m, 1H), 2.09-2.03 (m, 2H), 2.01 (s, 3H), 1.49 (d, 2H), 1.09 (d, 1H), 1.01 (t, 3H) |
| 1.11 | 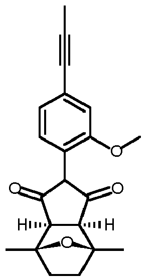 | Achiral | 1H NMR (400 MHz, CD₃OD) 7.12-7.08 (m, 1H), 6.96 (m, 2H), 3.76 (s, 3H), 2.85-2.75 (m, 2H), 2.02 (s, 3H), 1.85-1.78 (m, 2H), 1.70-1.63 (m, 2H), 1.51 (s, 6H) |
| 1.12 | 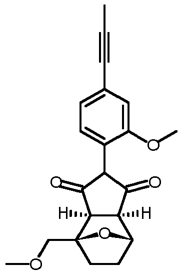 | Racemic | 1H NMR (400 MHz, CD₃OD) 7.09-7.02 (m, 1H), 6.97-6.88 (m, 2H), 4.61-4.49 (m, 1H), 3.88 (s, 1H), 3.77-3.74 (s, 3H), 3.74-3.63 (m, 1H), 3.40 (s, 3H), 2.95-2.91 (m, 1H), 2.85-2.78 (m, 1H), 2.03-2.01 (s, 3H), 2.00-1.83 (m, 2H), 1.74-1.66 (m, 1H), 1.63-1.55 (m, 1H) |
| 1.13 | 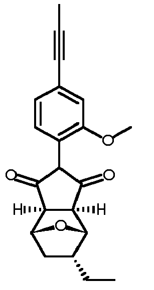 | Racemic | 1H NMR (400 MHz, CD₃OD) 7.07-7.03 (m, 1H), 6.93-6.89 (m, 2H), 4.54-4.50 (m, 1H), 4.47-4.44 (m, 1H), 3.72 (s, 3H), 2.96-2.92 (m, 1H), 2.67-2.64 (m, 1H), 2.09-2.03 (m, 2H), 2.01 (s, 3H), 1.49 (d, 2H), 1.09 (d, 1H), 1.01 (t, 3H) |
| 1.14 | 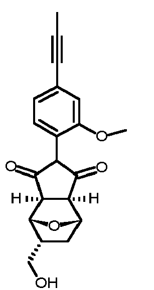 | Racemic | 1H NMR (400 MHz, CD₃OD) 7.06 (d, 1H), 6.96-6.90 (m, 2H), 4.59-4.51 (m, 2H), 3.77 (d, 1H), 3.74 (s, 3H), 3.66-3.55 (m, 1H), 3.15-3.09 (m, 1H), 2.76 (d, 1H), 2.50-2.38 (m, 1H), 2.04-1.96 (m, 4H), 1.20-1.09 (m, 1H) |
| 1.15 | 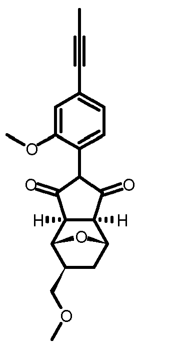 | Racemic | 1H NMR (400 MHz, CD₃OD) 7.11-6.91 (m, 3H), 4.58-4.52 (m, 1H), 4.47-4.41 (m, 1H), 3.73 (s, 3H), 3.33 (s, 3H), 3.24-3.11 (m, 2H), 2.83-2.75 (m, 2H), 2.21-2.12 (m, 1H), 2.01 (s, 3H), 1.79-1.69 (m, 1H), 1.34-1.25 (m, 1H) |
| 1.16 | 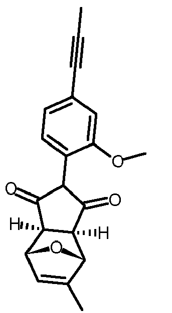 | Racemic | 1H NMR (400 MHz, CD₃OD) 7.08-7.03 (m, 1H), 6.97-6.91 (m, 2H), 6.07-5.96 (m, 1H), 4.94-4.82 (m, 1H), 4.74-4.61 (m, 1H), 3.74 (s, 3H), 2.87-2.70 (m, 2H), 2.02 (s, 3H), 1.93-1.86 (m, 3H) |
| 1.17 | 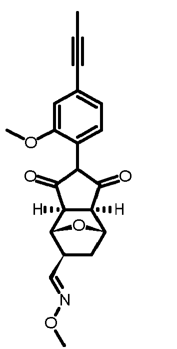 | Racemic | 1H NMR (400 MHz, CD₃OD) 7.30-7.23 (m, 1H), 6.95 (s, 2H), 4.72-4.54 (m, 2H), 3.90-3.87 (m, 1H), 3.77 (s, 3H), 3.74 (s, 3H), 2.91-2.82 (m, 3H), 2.78-2.71 (m, 1H), 2.01 (s, 3H), 1.64 - 1.94 (m, 2 H) |
| 1.18 | 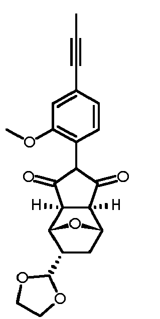 | Racemic | 1H NMR (400 MHz, CD₃OD) 7.10-7.04 (m, 1H), 6.96-6.91 (m, 2H), 4.87 (obscured, assume 1H d), 4.60-4.47 (m, 2H), 4.02-3.96 (m, 2H), 3.91-3.85 (m, 2H), 3.75-3.71 (s, 3H), 3.22-3.14 (m, 1H), 2.82-2.77 (m, 1H), 2.45-2.34 (m, 1H), 2.01 (s, 3H), 2.00-1.94 (m, 1H), 1.58-1.50 (m, 1H) |
| 1.19 | 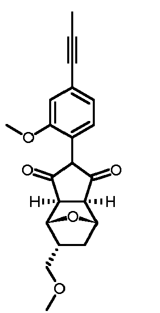 | ENT1 | 1HNMR (400 MHz, CD₃OD) 7.09-6.96 (m, 3H), 4.59-4.54 (m, 2H), 3.79-3.74 (s, 3H), 3.61-3.56 (m, 1H), 3.50-3.43 (s, 1H), 3.41-3.37 (m, 3H), 3.11-3.06 (m, 1H), 2.82-2.78 (m, 1H), 2.55-2.46 (m, 2H), 2.08-1.99 (m, 4H), 1.21-1.26 (m, 1H) |
| 1.20 | 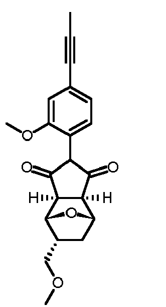 | ENT2 | 1HNMR (400 MHz, CD₃OD) 7.09-6.96 (m, 3H), 4.59-4.54 (m, 2H), 3.79-3.74 (s, 3H), 3.61-3.56 (m, 1H), 3.50-3.43 (s, 1H), 3.41-3.37 (m, 3H), 3.11-3.06 (m, 1H), 2.82-2.78 (m, 1H), 2.55-2.46 (m, 2H), 2.08-1.99 (m, 4H), 1.21-1.26 (m, 1H) |
| 1.21 | 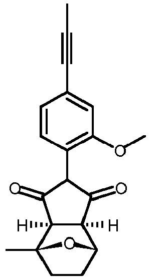 | ENT1 | 1H NMR (400 MHz, CD₃OD) 7.07 (d, 1H), 6.97-6.92 (m, 2H), 4.53 (d, 1H), 3.75 (s, 3H), 2.92 (d, 1H), 2.70 (d, 1H), 2.02 (s, 3H), 1.97-1.87 (m, 1H), 1.73 (d, 2H), 1.60-1.49 (m, 4H) |
| 1.22 | 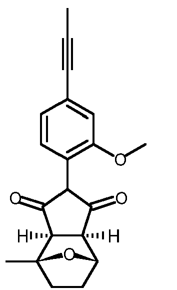 | ENT2 | 1H NMR (400 MHz, CD₃OD) 7.07 (d, 1H), 6.97 - 6.92 (m, 2H), 4.53 (d, 1H), 3.75 (s, 3H), 2.92 (d, 1H), 2.70 (d, 1H), 2.02 (s, 3H), 1.97-1.87 (m, 1H), 1.73 (d, 2H), 1.60-1.49 (m, 4H) |
| 1.23 | 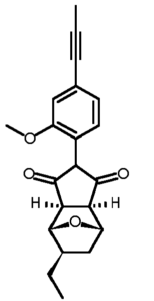 | ENT1 | 1H NMR (400 MHz, CD₃OD) 7.06-7.02 (m, 1H), 6.90-6.86 (m, 2H), 4.55 (d, 1H), 4.31 (s, 1H), 3.70 (s, 3 H), 2.55-2.52 (m, 2H), 2.00 (s, 3H), 1.84-1.70 (m, 2H), 1.56-1.17 (m, 3H), 0.93 (t, 3H) |
| 1.24 | 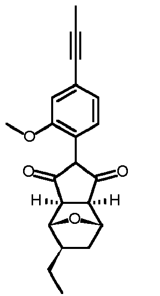 | ENT2 | 1H NMR (400 MHz, CD₃OD) 7.06-7.02 (m, 1H), 6.90-6.86 (m, 2H), 4.55 (d, 1H), 4.31 (s, 1H), 3.70 (s, 3H), 2.55-2.52 (m, 2H), 2.00 (s, 3H), 1.84-1.70 (m, 2H), 1.56-1.17 (m, 3H), 0.93 (t, 3H) |
| 1.25 | 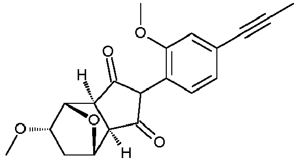 | Racemic | 1H NMR (400 MHz, CD₃OD) 7.09-7.04 (m, 1H), 6.98-6.91 (m, 2H), 4.58 (d, 1H), 4.54 (d, 1H), 3.95 (ddd, 1H), 3.75 (s, 3H), 3.39-3.36 (m, 3H), 3.34-3.31 (m, 1H), 2.87 (d, 1H), 2.18 (ddd, 1H), 2.02 (s, 3H), 1.36 (dd, 1H) |
| 1.26 | 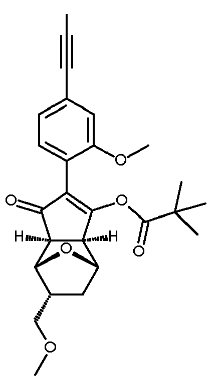 | Racemic | 1H NMR (400 MHz, CDCI3) 7.18-7.11 (m, 1H), 7.04-6.98 (m, 1H), 6.93-6.84 (m, 1H), 4.73-4.66 (m, 1H), 4.50-4.42 (m, 1H), 3.75-3.69 (m, 3H), 3.63-3.44 (m, 2H), 3.37-3.29 (m, 4H), 3.09-3.03 (m, 1H), 2.61-2.45 (m, 1H), 2.09-2.00 (m, 4H), 1.29-1.23 (m, 1H), 1.24-1.17 (m, 9H) |
| 1.27 | 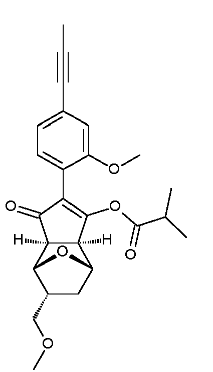 | Racemic | 1H NMR (400 MHz, CDCI3) 7.19-7.12 (m, 1H), 7.05-6.99 (m, 1H), 6.93-6.89 (m, 1H), 4.74-4.66 (m, 1H), 4.51-4.46 (m, 1H), 3.74-3.67 (m, 3H), 3.51-3.43 (m, 1H), 3.39-3.31 (m, 4H), 3.09-3.00 (m, 1H), 2.76-2.61 (m, 1H), 2.57-2.41 (m, 1H), 2.12-2.00 (m, 5H), 1.30-1.24 (m, 1H), 1.23-1.13 (m, 6H) |
| 1.28 | 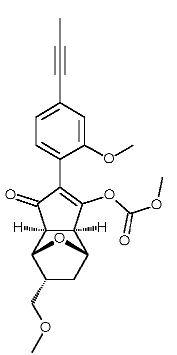 | Racemic | 1H NMR (400 MHz, CDCI3) 7.23-7.13 (m, 1H), 7.04-6.98 (m, 1H), 6.93-6.86 (m, 1H), 4.73-4.66 (m, 1H), 4.61-4.54 (m, 1H), 3.83-3.80 (m, 3H), 3.74-3.70 (m, 3H), 3.61-3.47 (m, 1H), 3.43-3.36 (m, 2H), 3.36-3.31 (m, 3H), 3.11-3.01 (m, 1H), 2.60-2.46 (m, 1H), 2.11-2.06 (m, 1H), 2.06-2.02 (m, 3H), 1.26-1.22 (m, 1H) |
| 1.29 | 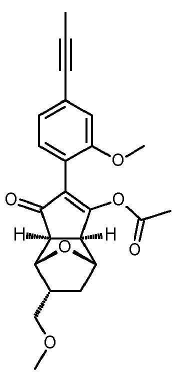 | Racemic | 1H NMR (400 MHz, CDCl3) 7.22-7.14 (m, 1H), 7.07-6.97 (m, 1H), 6.96-6.89 (m, 1H), 4.75-4.63 (m, 1H), 4.59-4.52 (m, 1H), 3.86-3.78 (m, 1H), 3.77 (s, 3H), 3.51-3.42 (m, 1H), 3.39-3.32 (m, 4H), 3.11-3.00 (m, 1H), 2.63-2.44 (m, 1H), 2.23-2.15 (m, 3H), 2.06-1.99 (m, 3H), 1.17-1.07 (m, 1H) |
| 1.30 | 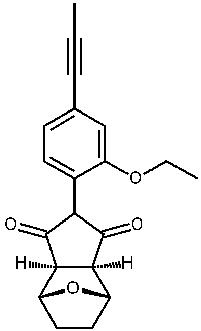 | Racemic | 1H NMR (400 MHz, CD₃OD) 7.07 (d, 1H), 6.91-6.90 (m, 2H), 4.58 (s, 2H), 3.96-3.93 (m, 2H), 2.71 (s, 2H), 1.99 (s, 3H), 1.78-1.76 |
| 1.31 | 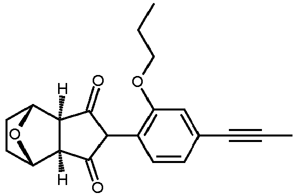 | Racemic | 1H NMR (400 MHz, DMSO-D6) 6.97 (d, 1H), 6.86-6.85 (m, 2H), 4.43 (s, 2H), 3.80 (t, 2H), 2.54 (s, 2H), 2.03 (s, 3H), 1.61-1.60 (m, 4H), 1.50-1.49 (m, 2H), 0.92 (t, 3H). |
| 1.32 | 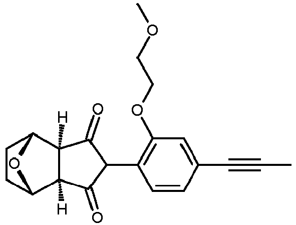 | Racemic | 1H NMR (400 MHz, DMSO-D6) 11.7 (bs, 1H), 7.0 (d, 1H), 6.95-6.91 (m, 2H), 4.47 (s, 2H), 4.01 (t, 2H), 3.57 (t, 2H), 3.27 (s, 3H), 2.69 (bs, 2H), 2.04 (s, 3H), 1.65-1.64 (m, 2H), 1.54-1.53 (m, 2H). |
| 1.33 | 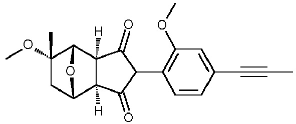 | Racemic | 1H NMR (400 MHz, DMSO-D6) 11.7 (bs, 1H), 7.0 (d, 1H), 6.95-6.91 (m, 2H), 4.47 (s, 2H), 4.01 (t, 2H), 3.57 (t, 2H), 3.27 (s, 3H), 2.69 (bs, 2H), 2.04 (s, 3H), 1.65-1.64 (m, 2H), 1.54-1.53 (m, 2H). |

### Biological Examples

Seeds of a variety of test species are sown in standard soil in pots (*Lolium perenne* (LOLPE), *Setaria faberi* (SETFA), *Alopecurus myosuroides* (ALOMY), *Echinochloa crus-galli* (ECHCG), *Avena fatua* (AVEFA)). After cultivation for one day (pre-emergence) or after 8 days cultivation (post-emergence) under controlled conditions in a glasshouse (at 24/16°C, day/night; 14 hours light; 65 % humidity), the plants are sprayed with an aqueous spray solution derived from the formulation of the technical active ingredient in acetone / water (50:50) solution containing 0.5% Tween 20 (polyoxyethelyene sorbitan monolaurate, CAS RN 9005-64-5). Compounds are applied at 250 g/h. The test plants are then grown in a glasshouse under controlled conditions in a glasshouse (at 24/16°C, day/night; 14 hours light; 65 % humidity) and watered twice daily. After 13 days for pre and post-emergence, the test is evaluated for the percentage damage caused to the plant. The biological activities are shown in the following table on a five point scale (5 = 80-100%; 4 = 60-79%; 3=40-59%; 2=20-39%; *1*=0-19%).

**TABLE B1**

| Compound | LOLPE | | SETFA | | ALOMY | | ECHCG | | AVEFA | |
|---|---|---|---|---|---|---|---|---|---|---|
| | PRE | POST | PRE | POST | PRE | POST | PRE | POST | PRE | POST |
| 1.1 | 5 | 5 | 5 | 5 | NT | NT | 5 | 5 | 5 | 5 |
| 1.2 | 5 | 5 | 5 | 5 | NT | NT | 5 | 5 | 5 | 5 |
| 1.3 | 5 | 5 | 5 | 5 | 5 | 5 | 5 | 5 | 5 | 5 |
| 1.5 | 5 | 5 | 5 | 5 | 5 | 5 | 5 | 5 | 5 | 5 |
| 1.6 | 5 | 5 | 5 | 5 | 5 | 2 | 5 | 5 | 3 | 5 |
| 1.9 | 5 | 5 | 5 | 5 | 5 | 5 | 5 | 5 | 5 | 5 |
| 1.11 | 4 | 5 | 3 | 5 | 5 | 5 | 3 | 5 | 3 | 5 |
| 1.12 | 5 | 5 | 5 | 5 | 5 | 5 | 5 | 5 | 5 | 5 |
| 1.14 | NT | 4 | NT | 5 | NT | 3 | NT | 5 | NT | 5 |
| 1.15 | 5 | 5 | 5 | 5 | 5 | 5 | 5 | 5 | 5 | 5 |
| 1.16 | 5 | 5 | 5 | 5 | 5 | 5 | 5 | 5 | 5 | 5 |
| 1.17 | 5 | 5 | 5 | 5 | 5 | 5 | 5 | 5 | 5 | 5 |
| 1.18 | 5 | 5 | 5 | 5 | 4 | 5 | 5 | 5 | 4 | 5 |
| 1.19 | 5 | 5 | 5 | 5 | 5 | 5 | 5 | 5 | 5 | 5 |
| 1.21 | 4 | 4 | 4 | 5 | 4 | 4 | 3 | 5 | 4 | 5 |
| 1.22 | 5 | 5 | 5 | 5 | 5 | 5 | 5 | 5 | 5 | 5 |
| 1.23 | 5 | 5 | 5 | 5 | 5 | 5 | 5 | 5 | 5 | 5 |
| 1.24 | 5 | 5 | 5 | 5 | 5 | 5 | 5 | 5 | 5 | 5 |
| 1.25 | 5 | 5 | 5 | 5 | 5 | 5 | 5 | 5 | 5 | 5 |
| 1.26 | 5 | 5 | 5 | 5 | 5 | 5 | 5 | 5 | 5 | 5 |
| 1.27 | 5 | 5 | 5 | 5 | 5 | 5 | 5 | 5 | 5 | 5 |
| 1.28 | 5 | 5 | 5 | 5 | 5 | 5 | 5 | 5 | 5 | 5 |
| 1.29 | 5 | 5 | 5 | 5 | 5 | 5 | 5 | 5 | 5 | 5 |
| 1.30 | 5 | 5 | 5 | NT | 5 | 5 | 5 | 5 | 5 | 5 |
| 1.31 | 5 | 5 | 5 | 5 | 5 | 5 | 5 | 5 | 5 | 5 |
| 1.32 | 4 | 5 | 3 | 5 | 1 | 4 | 3 | 5 | 4 | 5 |
| 1.33 | 5 | 5 | 5 | 5 | 5 | 5 | 5 | 5 | 5 | 5 |

| | | | | | | | | | | |
|---|---|---|---|---|---|---|---|---|---|---|
| NT = not tested. | | | | | | | | | | |

Using procedures outlines above, wheat plants (*Triticum aestivum* (TRZAW) are treated post-emergence with compound 1.2 of the present invention or compound A-18 from WO 2013/079708. The results outlined in Table B2 below show % phytotoxicity observed and that compound 1.2 of the present invention is less damaging to wheat compared to compound A-18 disclosed in WO2013/079708.

**TABLE B2**

| **Compound** | **Rate g/ha** | **TRZAW % Phyto** |
|---|---|---|
| Compound 1.2 | 63 | 60 |
| 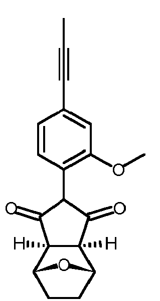 | 16 | 70 |
| | 4 | 20 |
| WO 2013/079708: A-18 | 63 | 100 |
| 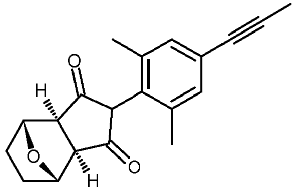 | 16 | 100 |
| | 4 | 60 |

**TABLE B3**

| | | |
|---|---|---|
| Using procedures outlines above, soybean plants (*Glycine max* (GLYMX) are treated post-emergence with compound 1.2 of the present invention or compound A-18 from WO 2013/079708. The results outlined in Table B3 below show % phytotoxicity observed and that compound 1.2 of the present invention is less damaging to soybean compared to compound A-18 disclosed in WO2013/079708. | | |

| **Compound** | **Rate g/ha** | **GLYMX % Phyto** |
|---|---|---|
| Compound 1.2 | 60 | 0 |
| 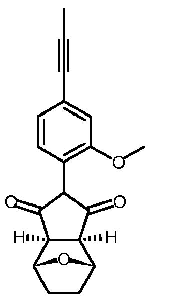 | 30 | 0 |
| | 15 | 0 |
| WO 2013/079708: A-18 | 60 | 80 |
| 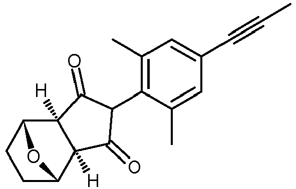 | 30 | 60 |
| | 15 | 40 |

## Claims

1. A compound of Formula (I): 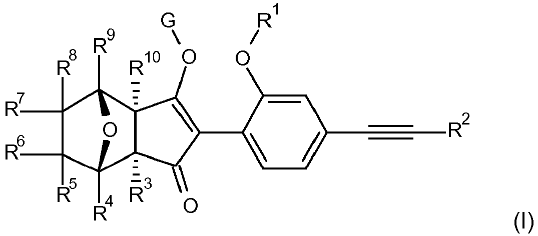 wherein
G is selected from the group consisting of hydrogen, -(CH₂)ₙ-R^{a}, -C(O)-R^{a}, - C(O)-O-R^{d}, -C(O)NR^{a}R^{a}, -S(O)₂-C₁-C₈alkyl and -C₁-C₃alkoxyC₁-C₈alkyl;
R^{a} is independently selected from the group consisting of hydrogen, C₁-C₈alkyl, C₁-C₃haloalkyl, C₂-C₈alkenyl, C₂-C₈alkynyl, C₃-C₆ cycloalkyl and phenyl;
R^{d} is independently selected from the group consisting of C₁-C₈alkyl, C₁-C₃haloalkyl, C₂-C₈alkenyl, C₂-C₈alkynyl, C₃-C₆ cycloalkyl and phenyl;
R¹ is selected from the group consisting of C₁-C₃alkyl, C₁-C₃alkoxyC₁-C₃alkyl- and C₁-C₃haloalkyl;
R² is C₁-C₃alkyl;
R³ and R¹⁰ are independently selected from the group consisting of hydrogen and C₁-C₃alkyl;
R⁴ and R⁹ are independently selected from the group consisting of hydrogen, C₁-C₃alkyl and C₁-C₃alkoxyC₁-C₃alkyl;
R⁶ and R⁷ are independently selected from the group consisting of hydrogen, halogen, -(CH₂)ₙ-OH, cyano, C₁-C₆alkyl, C₃-C₆cycloalkyl-, C₁-C₆haloalkyl, C₂-C₆alkenyl, C₂-C₆alkynyl, C₁-C₆alkoxy, C₂-C₆alkenyloxy-, C₂-C₆alkynyloxy-, C₁-C₆alkoxyC₁-C₆alkyl-, C₁-C₆alkoxyC₁-C₆alkoxy-, -O-C(O)C₁-C₆alkyl, - CH₂OCH₂CN, -CH=NOH, -CH=NO-C₁-C₃alkyl, -C(CH₃)=NOH, -C(CH₃)=NO-C₁-C₃alkyl, -CH₂OC(O)NHC₁-C₆alkyl, -(CH₂)ₙNR^{b}R^{c}, -C(O)NR^{b}R^{c}, - (CH₂)ₙNHC(O)H, -(CH₂)ₙNHC(O)C₁-C₆alkyl, -(CH₂)ₙNHC(O)OC₁-C₆alkyl, - NHC(O)NHC(O)C₁-C₆alkyl, -(CH₂)ₙ-N(R^{b})OR^{c}, -NHC(O)NR^{b}R^{c}, C₁-C₆haloalkoxy-, C₂-C₆alkenoxyC₁-C₆alkyl-, C₂-C₆alkynyloxyC₁-C₆alkyl-, C₁-C₆haloalkoxyC₁-C₆alkyl-, aryl, heteroaryl, and a 5 or 6-membered saturated or partially unsaturated ring system wherein the aryl, heteroaryl and ring system are optionally substituted by one or two independent R¹¹;
R^{b} and R^{c} are independently selected from the group consisting of hydrogen, phenyl and C₁-C₆alkyl; and
R⁵ and R⁸ form a bond or are independently selected from the group consisting of hydrogen, halogen, cyano, C₁-C₆alkyl, C₂-C₆alkenyl, C₂-C₆alkynyl, C₁-C₆alkoxy, C₁-C₆alkoxyC₁-C₆alkyl- and C₁-C₆alkoxyC₁-C₆alkoxy-; or
R⁵ and R⁶ together form =O, =NOH, =NOC₁-C₃alkyl, -X⁴-CH₂-CH₂-X⁵- or -X⁴-CH₂-CH₂-CH₂-X⁵- wherein X⁴ is CH₂ or O and X⁵ is CH₂, O or NH; and R⁷ and R⁸ are independently selected from the group consisting of hydrogen, halogen, cyano, C₁-C₆alkyl, C₂-C₆alkenyl, C₂-C₆alkynyl, C₁-C₆alkoxy-, C₁-C₆alkoxyC₁-C₆alkyl- and C₁-C₆alkoxyC₁-C₆alkoxy-; or
R⁷ and R⁸ together form =O, =NOH, =NOC₁-C₃alkyl, -X⁴-CH₂-CH₂-X⁵- or -X⁴-CH₂-CH₂-CH₂-X⁵- wherein X⁴ is CH₂ or O and X⁵ is CH₂, O or NH; and R⁵ and R⁶ are independently selected from the group consisting of hydrogen, halogen, cyano, C₁-C₆alkyl, C₂-C₆alkenyl, C₂-C₆alkynyl, C₁-C₆alkoxy, C₁-C₆alkoxyC₁-C₆alkyl- and C₁-C₆alkoxyC₁-C₆alkoxy-; and
R¹¹ is selected from the group consisting of C₁-C₃alkyl, C₁-C₃haloalkyl-, C₁-C₃alkoxy-, C₁-C₃haloalkoxy-, cyano and halogen; and
n = 0, 1 or 2;
or an agriculturally acceptable salt thereof.

2. A compound according to claim 1, wherein R¹ is methyl.

3. A compound according to any one of the previous claims, wherein R² is methyl.

4. A compound according to any one of claims 1 to 3, wherein R³, R⁴, R⁵, R⁶, R⁷, R⁸, R⁹ and R¹⁰ are hydrogen.

5. A compound according to any one of claims 1 to 3, wherein R³, R⁴, R⁵, R⁸, R⁹ and R¹⁰ are hydrogen and
R⁶ is hydrogen and R⁷ is methoxymethyl- or
R⁶ is methoxymethyl- and R⁷ is hydrogen.

6. A compound according to any one of claims 1 to 3, wherein R⁷ is selected from the group consisting of A1, A2 and A3: 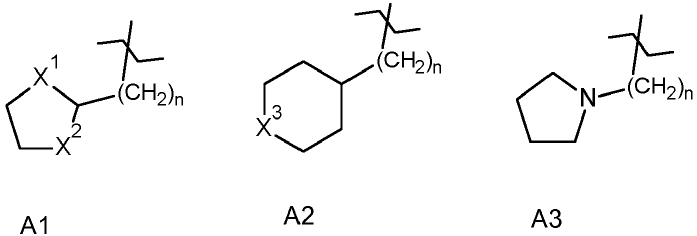 X¹, X² and X³ are independently selected from the group consisting of O, C(R¹²R¹³), N-(O-C₁-C₃alkyl), N-(CO)-C₁-C₃alkyl and N-(CO)O-C₁-C₃alkyl, wherein R¹² and R¹³ are independently hydrogen or C₁-C₆ alkyl.

7. A compound according to claim 6, wherein R⁷ is A1, X¹ and X² are oxygen and n is 0.

8. A compound according to any one of claims 1 to 3, wherein R⁵ and R⁸ form a bond and R³, R⁴, R⁶, R⁷, R⁹ and R¹⁰ are hydrogen.

9. A compound according to any one of the previous claims, wherein G is hydrogen or -C(O)C₁-C₆alkyl.

10. A compound according to any one of the previous claims, wherein G is hydrogen.

11. A herbicidal composition comprising a compound according to any one of the previous claims and an agriculturally acceptable formulation adjuvant.

12. A herbicidal composition according to claim 11, further comprising at least one additional pesticide.

13. A herbicidal composition according to claim 12, wherein the additional pesticide is a herbicide or herbicide safener.

14. A method of controlling weeds at a locus comprising application to the locus of a weed controlling amount of a composition according to any one of claims 11 to 13.

15. Use of a compound of Formula (I) as defined in claim 1 as a herbicide.

## Patentansprüche

1. Verbindung der Formel (I): 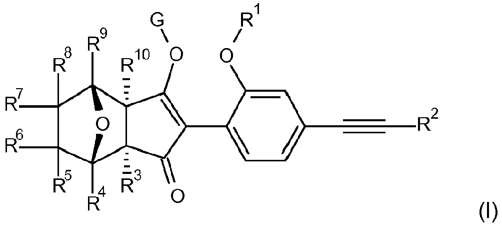 wobei
G aus der Gruppe bestehend aus Wasserstoff, - (CH₂)ₙ-R^{a}, -C(O)-R^{a}, -C(O)-O-R^{d}, -C(O)NR^{a}R^{a}, -S(O)₂-C₁-C₈-Alkyl und -C₁-C₃-Alkoxy-C₁-C₈-alkyl ausgewählt ist;
R^{a} unabhängig aus der Gruppe bestehend aus Wasserstoff, C₁-C₈-Alkyl, C₁-C₃- Halogenalkyl, C₂-C₈-Alkenyl, C₂-C₈-Alkinyl, C₃-C₆-Cycloalkyl und Phenyl ausgewählt ist;
R^{d} unabhängig aus der Gruppe bestehend aus C₁-C₈-Alkyl, C₁-C₃-Halogenalkyl, C₂-C₈-Alkenyl, C₂-C₈-Alkinyl, C₃-C₆-Cycloalkyl und Phenyl ausgewählt ist;
R¹ aus der Gruppe bestehend aus C₁-C₃-Alkyl, C₁-C₃-Alkoxy-C₁-C₃-alkyl und C₁-C₃-Halogenalkyl ausgewählt ist;
R² für C₁-C₃-Alkyl steht;
R³ und R¹⁰ jeweils unabhängig aus der Gruppe bestehend aus Wasserstoff und C₁-C₃-Alkyl ausgewählt sind;
R⁴ und R⁹ jeweils unabhängig aus der Gruppe bestehend aus Wasserstoff, C₁-C₃-Alkyl und C₁-C₃-Alkoxy-C₁-C₃-alkyl ausgewählt sind;
R⁶ und R⁷ unabhängig aus der Gruppe bestehend aus Wasserstoff, Halogen, - (CH₂)ₙ-OH, Cyano, C₁-C₆-Alkyl, C₃-C₆-Cycloalkyl-, C₁-C₆-Halogenalkyl, C₂-C₆-Alkenyl, C₂-C₆-Alkinyl, C₁-C₆-Alkoxy, C₂-C₆-Alkenyloxy-, C₂-C₆-Alkinyloxy-, C₁-C₆-Alkoxy-C₁-C₆-alkyl-, C₁-C₆-Alkoxy-C₁-C₆-alkoxy-, -O-C(O)-C₁-C₆-Alkyl, -CH₂OCH₂CN, -CH=NOH, -CH=NO-C₁-C₃-Alkyl, -C(CH₃)=NOH, -C(CH₃)=NO-C₁-C₃-Alkyl, -CH₂OC(O)NH-C₁-C₆-Alkyl, - (CH₂)ₙNR^{b}R^{c}, -C(O)NR^{b}R^{c}, -(CH₂)ₙNHC(O)H, - (CH₂)ₙNHC(O)-C₁-C₆-Alkyl, -(CH₂)ₙNHC(O)O-C₁-C₆-Alkyl, -NHC(O)NHC (O)-C₁-C₆-Alkyl, -(CH₂)ₙ-N(R^{b})OR^{c}, -NHC(O)NR^{b}R^{c}, C₁-C₆-Halogenalkoxy, C₂-C₆-Alkenoxy-C₁-C₆-alkyl-, C₂-C₆-Alkinyloxy-C₁-C₆-alkyl-, C₁-C₆-Halogenalkoxy-C₁-C₆-alkyl, Aryl, Heteroaryl und einem 5- oder 6-gliedrigen gesättigten oder teilweise ungesättigten Ringsystem ausgewählt sind, wobei das Aryl, Heteroaryl und Ringsystem gegebenenfalls durch ein oder zwei unabhängige R¹¹ substituiert sind;
R^{b} und R^{c} unabhängig aus der Gruppe bestehend aus Wasserstoff, Phenyl und C₁-C₆-Alkyl ausgewählt sind; und
R⁵ und R⁸ eine Bindung bilden oder unabhängig aus der Gruppe bestehend aus Wasserstoff, Halogen, Cyano, C₁-C₆-Alkyl, C₂-C₆-Alkenyl, C₂-C₆-Alkinyl, C₁-C₆-Alkoxy, C₁-C₆-Alkoxy-C₁-C₆-alkyl- und C₁-C₆-Alkoxy-C₁-C₆-alkoxy- ausgewählt sind; oder
R⁵ und R⁶ zusammen =O, =NOH, =NO-C₁-C₃-Alkyl, -X⁴-CH₂-CH₂-X⁵- oder -X⁴-CH₂-CH₂-CH₂-X⁵- bilden, wobei X⁴ für CH₂ oder O steht und X⁵ für CH₂, O oder NH steht; und R⁷ und R⁸ unabhängig aus der Gruppe bestehend aus Wasserstoff, Halogen, Cyano, C₁-C₆-Alkyl, C₂-C₆-Alkenyl, C₂-C₆-Alkinyl, C₁-C₆-Alkoxy-, C₁-C₆-Alkoxy-C₁-C₆-alkyl- und C₁-C₆-Alkoxy-C₁-C₆-alkoxy- ausgewählt sind; oder
R⁷ und R⁸ zusammen =O, =NOH, =NO-C₁-C₃-Alkyl, -X⁴-CH₂-CH₂-X⁵- oder -X⁴-CH₂-CH₂-CH₂-X⁵- bilden, wobei X⁴ für CH₂ oder O steht und X⁵ für CH₂, O oder NH steht; und R⁵ und R⁶ unabhängig aus der Gruppe bestehend aus Wasserstoff, Halogen, Cyano, C₁-C₆-Alkyl, C₂-C₆-Alkenyl, C₂-C₆-Alkinyl, C₁-C₆-Alkoxy, C₁-C₆-Alkoxy-C₁-C₆-alkyl- und C₁-C₆-Alkoxy-C₁-C₆-alkoxy- ausgewählt sind; und
R¹¹ aus der Gruppe bestehend aus C₁-C₃-Alkyl, C₁-C₃-Halogenalkyl, C₁-C₃-Alkoxy-, C₁-C₃-Halogenalkoxy-, Cyano und Halogen ausgewählt ist; und
n = 0, 1 oder 2;
oder ein landwirtschaftlich unbedenkliches Salz davon.

2. Verbindung nach Anspruch 1, wobei R¹ für Methyl steht.

3. Verbindung nach einem der vorhergehenden Ansprüche, wobei R² für Methyl steht.

4. Verbindung nach einem der Ansprüche 1 bis 3, wobei R³, R⁴, R⁵, R⁶, R⁷, R⁸, R⁹ und R¹⁰ für Wasserstoff stehen.

5. Verbindung nach einem der Ansprüche 1 bis 3, wobei R³, R⁴, R⁵, R⁸, R⁹ und R¹⁰ für Wasserstoff stehen und R⁶ für Wasserstoff steht und R⁷ für Methoxymethylsteht oder
R⁶ für Methoxymethyl- steht und R⁷ für Wasserstoff steht.

6. Verbindung nach einem der Ansprüche 1 bis 3, wobei R⁷ aus der Gruppe bestehend aus A1, A2 und A3 ausgewählt ist: 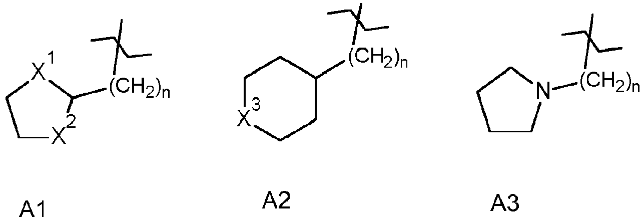 X¹, X² und X³ unabhängig aus der Gruppe bestehend aus O, C(R¹²R¹³), -N(O-C₁-C₃-Alkyl), -N-(CO)-C₁-C₃-Alkyl und -N-(CO)O-C₁-C₃-Alkyl ausgewählt sind, wobei R¹² und R¹³ unabhängig für Wasserstoff oder C₁-C₆-Alkyl stehen.

7. Verbindung nach Anspruch 6, wobei R⁷ für A1 steht, X¹ und X² für Sauerstoff stehen und n für 0 steht.

8. Verbindung nach einem der Ansprüche 1 bis 3, wobei R⁵ und R⁸ eine Bindung bilden und R³, R⁴, R⁶, R⁷, R⁹ und R¹⁰ für Wasserstoff stehen.

9. Verbindung nach einem der vorhergehenden Ansprüche, wobei G für Wasserstoff oder -C(O)-C₁-C₆-Alkyl steht.

10. Verbindung nach einem der vorhergehenden Ansprüche, wobei G für Wasserstoff steht.

11. Herbizide Zusammensetzung, umfassend eine Verbindung nach einem der vorhergehenden Ansprüche und ein landwirtschaftlich unbedenkliches Formulierungshilfsmittel.

12. Herbizide Zusammensetzung nach Anspruch 11, ferner umfassend mindestens ein zusätzliches Pestizid.

13. Herbizide Zusammensetzung nach Anspruch 12, wobei es sich bei dem zusätzlichen Pestizid um ein Herbizid oder einen Herbizid-Safener handelt.

14. Verfahren zur Bekämpfung von Unkräutern an einem Standort, bei dem man eine unkrautbekämpfende Menge einer Zusammensetzung nach einem der Ansprüche 11 bis 13 auf den Standort ausbringt.

15. Verwendung einer Verbindung der Formel (I) gemäß Anspruch 1 als Herbizid.

## Revendications

1. Composé de formule (I) 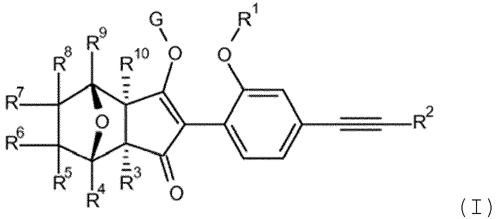 dans lequel
G est choisi dans le groupe constitué d'hydrogène, -(CH₂)ₙ-R^{a}, -C(O)-R^{a}, -C(O)-OR^{d}, -C(O)NR^{a}R^{a}, -S(O)₂-(alkyle en C₁-C₈) et -(alcoxy en C₁-C₃) - (alkyle en C₁-C₈) ;
R^{a} est indépendamment choisi dans le groupe constitué d'hydrogène, alkyle en C₁-C₈, halogénoalkyle en C₁-C₃, alcényle en C₂-C₈, alcynyle en C₂-C₈, cycloalkyle en C₃-C₆ et phényle ;
R^{d} est indépendamment choisi dans le groupe constitué d'alkyle en C₁-C₈, halogénoalkyle en C₁-C₃, alcényle en C₂-C₈, alcynyle en C₂-C₈, cycloalkyle en C₃-C₆ et phényle ; R¹ est choisi dans le groupe constitué d'alkyle en C₁-C₃, (alcoxy en C₁-C₃)-(alkyle en C₁-C₃)- et halogénoalkyle en C₁-C₃ ;
R² est alkyle en C₁-C₃ ;
R³ et R¹⁰ sont indépendamment choisis dans le groupe constitué d'hydrogène et alkyle en C₁-C₃ ;
R⁴ et R⁹ sont indépendamment choisis dans le groupe constitué d'hydrogène, alkyle en C₁-C₃ et (alcoxy en C₁-C₃)-(alkyle en C₁-C₃);
R⁶ et R⁷ sont indépendamment choisis dans le groupe constitué d'hydrogène, halogène, -(CH₂)ₙ-OH, cyano, alkyle en C₁-C₆, (cycloalkyle en C₃-C₆) -, halogénoalkyle en C₁-C₆, alcényle en C₂-C₆, alcynyle en C₂-C₆, alcoxy en C₁-C₆, (alcényloxy en C₂-C₆) -, (alcynyloxy en C₂-C₆) -, (alcoxy en C₁-C₆) - (alkyle en C₁-C₆) -, (alcoxy en C₁-C₆) - (alcoxy en C₁-C₆) -, -O-C(O)-(alkyle en C₁-C₆) , -CH₂OCH₂CN, -CH=NOH, -CH=NO-(alkyle en C₁-C₃), -C(CH₃)=NOH, -C(CH₃)=NO- (alkyle en C₁-C₃), -CH₂OC (O)NH (alkyle en C₁-C₆), - (CH₂)ₙNR^{b}R^{c}, -C(O)NR^{b}R^{c}, - (CH₂)ₙNHC(O)H, -(CH₂)ₙNHC(O)-(alkyle en C₁-C₆), - (CH₂)ₙNHC(O)O(alkyle en C₁-C₆), -NHC (O)NHC(O)-(alkyle en C₁-C₆) , -(CH₂)ₙ-N(R^{b})OR^{c}, -NHC(O)NR^{b}R^{c}, (halogénoalcoxy en C₁-C₆)-, (alcényloxy en C₂-C₆) - (alkyle en C₁-C₆)-, (alcynyloxy en C₂-C₆) - (alkyle en C₁-C₆) -, (halogénoalcoxy en C₁-C₆)- (alkyle en C₁-C₆)-, aryle, hétéroaryle, et un système cyclique saturé ou partiellement insaturé de 5 ou 6 chaînons dans lequel l'aryle, l'hétéroaryle et le système cyclique sont facultativement substitués par un ou deux R¹¹ indépendants ;
R^{b} et R^{c} sont indépendamment choisis dans le groupe constitué d'hydrogène, phényle et alkyle en C₁-C₆ ; et R⁵ et R⁸ forment une liaison ou sont indépendamment choisis dans le groupe constitué d'hydrogène, halogène, cyano, alkyle en C₁-C₆, alcényle en C₂-C₆, alcynyle en C₂-C₆, alcoxy en C₁-C₆, (alcoxy en C₁-C₆) -(alkyle en C₁-C₆)- et (alcoxy en C₁-C₆)-(alcoxy en C₁-C₆)- ; ou
R⁵ et R⁶ forment conjointement =O, =NOH, =NO(alkyle en C₁-C₃), -X⁴-CH₂-CH₂-X⁵- ou -X⁴-CH₂-CH₂-CH₂-X⁵- où X⁴ est CH₂ ou O et X⁵ est CH₂, O ou NH; et R⁷ et R⁸ sont indépendamment choisis dans le groupe constitué d'hydrogène, halogène, cyano, alkyle en C₁-C₆, alcényle en C₂-C₆, alcynyle en C₂-C₆, (alcoxy en C₁-C₆)-, (alcoxy en C₁-C₆)-(alkyle en C₁-C₆) - et (alcoxy en C₁-C₆)-(alcoxy en C₁-C₆)- ; ou
R⁷ et R⁸ forment conjointement =O, =NOH, =NO(alkyle en C₁-C₃), -X⁴-CH₂-CH₂-X⁵- ou -X⁴-CH₂-CH₂-CH₂-X⁵- où X⁴ est CH₂ ou O et X⁵ est CH₂, O ou NH ; et R⁵ et R⁶ sont indépendamment choisis dans le groupe constitué d'hydrogène, halogène, cyano, alkyle en C₁-C₆, alcényle en C₂-C₆, alcynyle en C₂-C₆, alcoxy en C₁-C₆, (alcoxy en C₁-C₆)-(alkyle en C₁-C₆)- et (alcoxy en C₁-C₆)-(alcoxy en C₁-C₆)- ; et
R¹¹ est choisi dans le groupe constitué d'alkyle en C₁-C₃, (halogénoalkyle en C₁-C₃)-, (alcoxy en C₁-C₃)-, (halogénoalcoxy en C₁-C₃)-, cyano et halogène ; et
n = 0, 1 ou 2 ;
ou un sel acceptable en agriculture de celui-ci.

2. Composé selon la revendication 1, dans lequel R¹ est méthyle.

3. Composé selon l'une quelconque des revendications précédentes, dans lequel R² est méthyle.

4. Composé selon l'une quelconque des revendications 1 à 3, dans lequel R³, R⁴, R⁵, R⁶, R⁷, R⁸, R⁹ et R¹⁰ sont hydrogène.

5. Composé selon l'une quelconque des revendications 1 à 3, dans lequel R³, R⁴, R⁵, R⁸, R⁹ et R¹⁰ sont hydrogène et
R⁶ est hydrogène et R⁷ est méthoxyméthyl- ou
R⁶ est méthoxyméthyl- et R⁷ est hydrogène.

6. Composé selon l'une quelconque des revendications 1 à 3, dans lequel R⁷ est choisi dans le groupe constitué de A1, A2 et A3 : 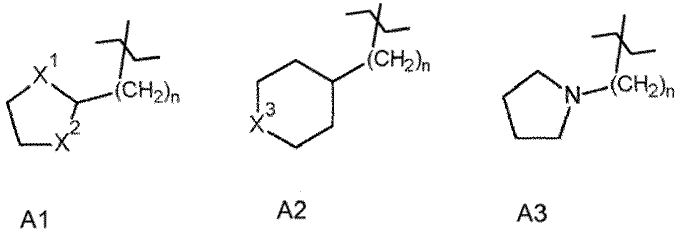 X¹, X² et X³ sont indépendamment choisis dans le groupe constitué de O, C(R¹²R¹³), N-(O-(alkyle en C₁-C₃)), N-(CO)-(alkyle en C₁-C₃) et N-(CO)O-(alkyle en C₁-C₃), où R¹² et R¹³ sont indépendamment hydrogène ou alkyle en C₁-C₆.

7. Composé selon la revendication 6, dans lequel R⁷ est A1, X¹ et X² sont oxygène et n est 0.

8. Composé selon l'une quelconque des revendications 1 à 3, dans lequel R⁵ et R⁸ forment une liaison et R³, R⁴, R⁶, R⁷, R⁹ et R¹⁰ sont hydrogène.

9. Composé selon l'une quelconque des revendications précédentes, dans lequel G est hydrogène ou -C(O)-(alkyle en C₁-C₆).

10. Composé selon l'une quelconque des revendications précédentes, dans lequel G est hydrogène.

11. Composition herbicide comprenant un composé selon l'une quelconque des revendications précédentes et un adjuvant de formulation acceptable en agriculture.

12. Composition herbicide selon la revendication 11, comprenant en outre au moins un pesticide supplémentaire.

13. Composition herbicide selon la revendication 12, dans laquelle le pesticide supplémentaire est un herbicide ou un phytoprotecteur.

14. Procédé de lutte contre les mauvaises herbes à un site comprenant l'application sur le site d'une quantité désherbante d'une composition selon l'une quelconque des revendications 11 à 13.

15. Utilisation d'un composé de formule (I) tel que défini dans la revendication 1 en tant qu'herbicide.
